(19) Europäisches Patentamt
European Patent Office
Office européen des brevets

(11) EP 4 700 023 A1

(12) **EUROPEAN PATENT APPLICATION**
published in accordance with Art. 153(4) EPC

(43) Date of publication:
**25.02.2026 Bulletin 2026/09**

(21) Application number: **24792137.2**

(22) Date of filing: **19.04.2024**

(51) International Patent Classification (IPC):
*C07D 405/14* (2006.01) *A61K 31/4439* (2006.01)
*A61P 3/00* (2006.01) *A61P 1/16* (2006.01)
*A61P 3/10* (2006.01) *A61P 3/06* (2006.01)
*A61P 3/04* (2006.01) *A61P 9/10* (2006.01)

(52) Cooperative Patent Classification (CPC):
**A61K 31/4439; A61K 31/454; A61P 1/16; A61P 3/00; A61P 3/04; A61P 3/06; A61P 3/08; A61P 3/10; A61P 9/10; A61P 9/12; C07C 213/10; C07C 215/10; C07D 319/12; C07D 405/14; C30B 29/54**

(86) International application number:
**PCT/CN2024/088887**

(87) International publication number:
**WO 2024/217553 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.04.2023 CN 202310435501**

(71) Applicant: **Chengdu Vincentage Pharma Co., Ltd.**
**Chengdu, Sichuan 610041 (CN)**

(72) Inventor: **LI, Ben**
**Suzhou, Jiangsu 215347 (CN)**

(74) Representative: **Dehns**
**10 Old Bailey**
**London EC4M 7NG (GB)**

(54) **GLP-1R RECEPTOR AGONIST COMPOUND SALT AND CRYSTAL FORM THEREOF, PREPARATION METHOD THEREFOR AND USE THEREOF**

(57) Disclosed in the present invention are a GLP-1R receptor agonist compound salt and a crystal form thereof, a preparation method therefor and a use thereof. Specifically, disclosed is a 2-amino-2-(hydroxymethyl)-1,3-propanediol salt of a compound of formula I or a solvate thereof. The 2-amino-2-(hydroxymethyl)-1,3-propanediol salt of the compound of formula I or the solvate thereof provided by the present invention has good crystal form stability and good medicinal prospects.

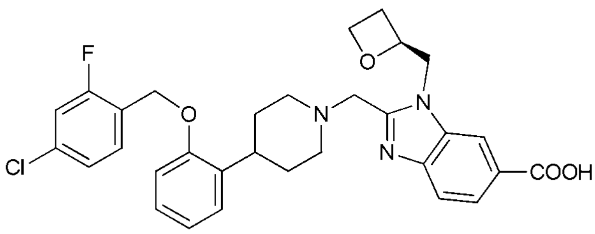

I

EP 4 700 023 A1

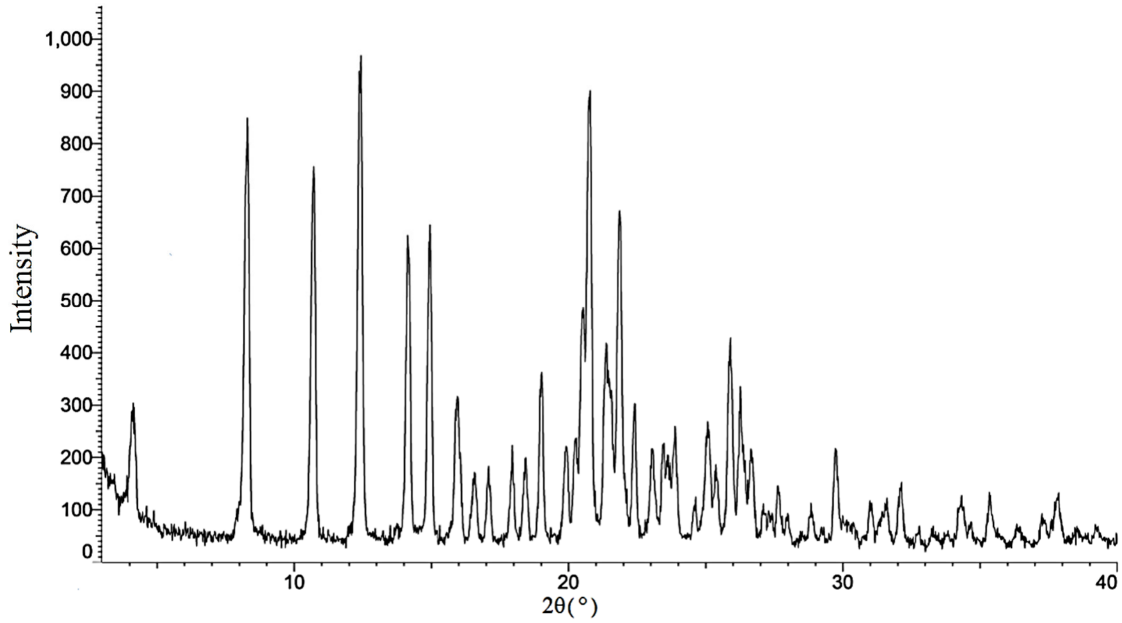
Intensity
1,000
900
800
700
600
500
400
300
200
100
0
10
20
30
40
2θ(°)

FIG. 1

## Description

The present application claims the priority of Chinese patent application 2023104355011 filed on April 21, 2023. The contents of the above Chinese patent application are incorporated herein by reference in its entirety.

### TECHNICAL FIELD

The present disclosure relates to a GLP-1R receptor agonist compound salt, a crystal form thereof, a preparation method therefor, and a use thereof, specifically to a 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate, a preparation method therefor, and a use thereof.

### BACKGROUND

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid is a GLP-1R agonist used for treating metabolism-related diseases such as diabetes, obesity, non-alcoholic fatty liver disease, and hypertension.

Glucagon-like peptide-1 (GLP-1) is an endogenous incretin that can stimulate insulin release. It binds to and activates the GLP-1 receptor (GLP-1R) of the class B G protein-coupled receptor (GPCR) family to exert its regulatory functions. GLP-1 receptor agonists (GLP-1RAs) are synthetic drugs used to mimic the physiological functions of GLP-1. After binding to the GLP-1R of pancreatic cells, GLP-1RAs can glucose-dependently stimulate insulin synthesis and secretion, reduce glucagon release, act on the GLP-1R in the central nervous system to decrease food intake, increase energy expenditure by promoting thermogenesis in brown adipose tissue and lipolysis in white adipose tissue, and delay gastric emptying. The GLP-1RA is clinically suitable for adult patients with T2DM and has a significant glucose-lowering effect while concurrently reducing body weight and systolic blood pressure, as well as improving dyslipidemia. The GLP-1RA can be used alone or in combination with other glucose-lowering drugs. Results from multiple clinical studies show that adding GLP-1RAs after the failure of a single oral glucose-lowering drug (metformin, sulfonylureas) is effective.

The preparation of the compound of formula I is described in Example 3 of the Chinese patent CN202110334388, submitted on March 29, 2021. The structure of the compound of formula I is as follows:

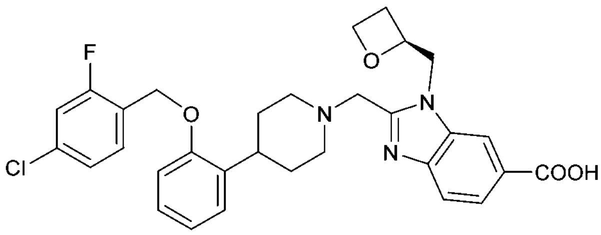

Compound of formula I

wherein the compound of formula I is (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylic acid, and the preparation method of the compound of formula I is prepared according to Example 3 of CN202110334388.

The compound of formula I is used for treating metabolism-related diseases. The metabolism-related diseases are selected from any one of glucose intolerance, hyperglycemia, dyslipidemia, type 1 diabetes (T1DM), type 2 diabetes (T2DM), hypertriglyceridemia, insulin resistance, impaired glucose tolerance (IGT), diabetic dyslipidemia, hyperlipidemia, arteriosclerosis, atherosclerosis, hypertension, obesity, non-alcoholic fatty liver disease, non-alcoholic steatohepatitis, liver fibrosis, cirrhosis, lethargy, and the like. However, the compound of formula I has poor stability under room temperature or high-temperature conditions, and has unsatisfactory solid properties. Under conditions such as grinding, compressing, or mixing of the compound, the stability and crystal form of the compound are prone to change. Therefore, there is an urgent need to improve the properties of compounds, as well as the stability and medicinal processability of compounds through novel approaches, and lay a solid foundation for the subsequent application of compounds.

### SUMMARY

The technical problem to be solved by the present disclosure is that the existing GLP-1R receptor agonist compounds are less of solid forms and are poor in stability. To this end, the present disclosure provides GLP-1R receptor agonist compound salts, crystal forms thereof, preparation methods therefor, and uses thereof. The crystal forms of the salts provided by the present disclosure exhibit good stability and possess promising medicinal prospects.

The present disclosure provides a 2-amino-2-(hydroxymethyl)-1,3-propanediol salt of a compound of formula I or a solvate thereof (a solvate of the 2-amino-2-(hydroxymethyl)-1,3-propanediol salt of the compound of formula I);

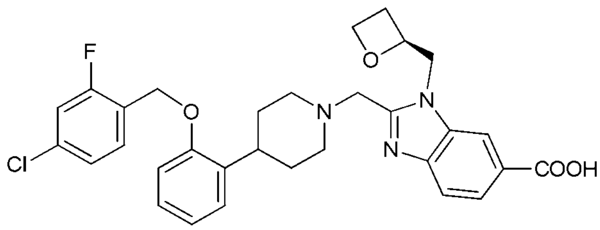

I

.

In some embodiments, the 2-amino-2-(hydroxymethyl)-1,3-propanediol salt of the compound of formula I is a compound of formula II,

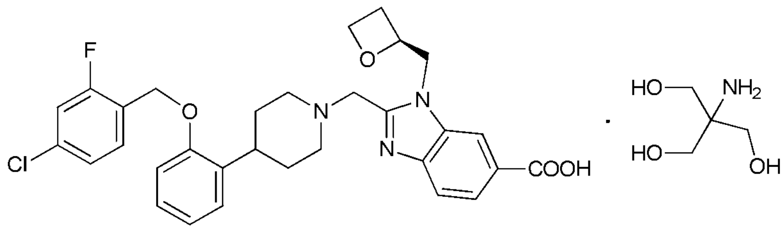

II

.

In some embodiments, in the solvate of the 2-amino-2-(hydroxymethyl)-1,3-propanediol salt of the compound of formula I, the solvent is water or 1,4-dioxane; in the solvate of the 2-amino-2-(hydroxymethyl)-1,3-propanediol salt of the compound of formula I, the molar ratio of the 2-amino-2-(hydroxymethyl)-1,3-propanediol salt of the compound of formula I to the solvent is preferably 1:(1 to 0.5), for example, the molar ratio of the 2-amino-2-(hydroxymethyl)-1,3-propanediol salt of the compound of formula I to water is 1:1, and for another example, the molar ratio of the 2-amino-2-(hydroxymethyl)-1,3-propanediol salt of the compound of formula I to 1,4-dioxane is 1:0.5.

The present disclosure provides a crystal form A of a monohydrate of a compound of formula II, which has an X-ray powder diffraction pattern obtained using Cu-Kα radiation and expressed in terms of 20 angles, comprising diffraction peaks at 4.1±0.2°, 8.2±0.2°, 10.7±0.2°, 12.3±0.2°, and 20.7±0.2°;

the structure of the monohydrate of the compound of formula II is:

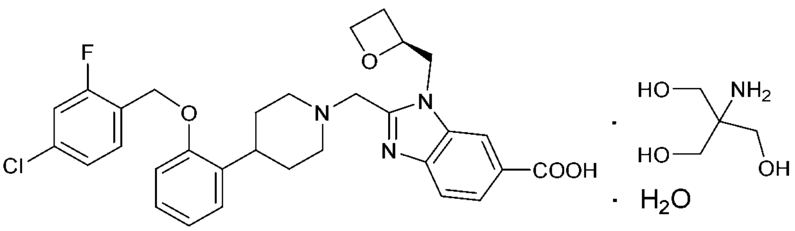

.

Preferably, the single crystal of the monohydrate of the compound of formula II is orthorhombic.

In some embodiments, the X-ray powder diffraction pattern of the crystal form A of the monohydrate of the compound of formula II, obtained using Cu-Kα radiation and expressed in terms of 20 angles, further comprises diffraction peaks at one or more of the following positions: 14.1±0.2°, 14.9±0.2°, 20.5±0.2°, and 21.8±0.2°.

In some embodiments, the X-ray powder diffraction pattern of the crystal form A of the monohydrate of the compound of formula II, obtained using Cu-Kα radiation and expressed in terms of 20 angles, comprises diffraction peaks at 4.1±0.2°, 8.2±0.2°, 10.7±0.2°, 12.3±0.2°, 14.1±0.2°, 14.9±0.2°, 20.5±0.2°, and 21.8±0.2°.

In some embodiments, the X-ray powder diffraction pattern of the crystal form A of the monohydrate of the compound of formula II, obtained using Cu-Kα radiation and expressed in terms of 2θ angles, comprises diffraction peaks as shown in Table 1:

Table 1

| Number | 2θ angle | d | Relative peak intensity |
|---|---|---|---|
| 1 | 4.114° | 21.46041 Å | 19.8% |
| 2 | 8.295° | 10.65090 Å | 91.1% |
| 3 | 10.703° | 8.25895 Å | 80.6% |
| 4 | 12.388° | 7.13911 Å | 100.0% |
| 5 | 14.151° | 6.25379 Å | 64.9% |
| 6 | 14.941° | 5.92472 Å | 65.2% |
| 7 | 15.954° | 5.55077 Å | 31.3% |
| 8 | 16.566° | 5.34683 Å | 13.9% |
| 9 | 17.086° | 5.18547 Å | 16.0% |
| 10 | 17.948° | 4.93836 Å | 18.9% |
| 11 | 18.429° | 4.81041 Å | 17.4% |
| 12 | 19.019° | 4.66256 Å | 36.0% |
| 13 | 19.916° | 4.45440 Å | 19.7% |
| 14 | 20.248° | 4.38230 Å | 21.0% |
| 15 | 20.530° | 4.32259 Å | 48.4% |
| 16 | 20.768° | 4.27354 Å | 94.2% |
| 17 | 21.362° | 4.15615 Å | 40.2% |
| 18 | 21.868° | 4.06113 Å | 70.7% |
| 19 | 22.405° | 3.96502 Å | 28.4% |
| 20 | 23.059° | 3.85389 Å | 18.2% |
| 21 | 23.528° | 3.77811 Å | 12.6% |
| 22 | 23.874° | 3.72426 Å | 22.6% |
| 23 | 24.612° | 3.61423 Å | 5.5% |
| 24 | 25.081° | 3.54767 Å | 23.8% |
| 25 | 25.383° | 3.50611 Å | 14.5% |
| 26 | 25.899° | 3.43736 Å | 42.5% |
| 27 | 26.264° | 3.39049 Å | 30.7% |
| 28 | 26.667° | 3.34010 Å | 18.2% |
| 29 | 27.176° | 3.27878 Å | 4.8% |
| 30 | 27.646° | 3.22405 Å | 11.1% |
| 31 | 27.968° | 3.18764 Å | 4.4% |
| 32 | 28.847° | 3.09251 Å | 7.2% |
| 33 | 29.734° | 3.00219 Å | 20.1% |
| 34 | 30.268° | 2.95045 Å | 2.4% |
| 35 | 30.988° | 2.88351 Å | 8.4% |
| 36 | 31.535° | 2.83476 Å | 8.1% |
| 37 | 32.098° | 2.78633 Å | 11.5% |
| 38 | 32.770° | 2.73066 Å | 2.7% |
| 39 | 33.246° | 2.69269 Å | 1.9% |

(continued)

| Number | 2θ angle | d | Relative peak intensity |
|---|---|---|---|
| 40 | 34.317° | 2.61104 Å | 9.7% |
| 41 | 34.659° | 2.58607 Å | 4.3% |
| 42 | 35.356° | 2.53667 Å | 9.7% |
| 43 | 36.387° | 2.46713 Å | 2.7% |
| 44 | 37.305° | 2.40846 Å | 4.9% |
| 45 | 37.817° | 2.37704 Å | 9.3% |
| 46 | 38.537° | 2.33425 Å | 2.8% |
| 47 | 39.257° | 2.29310 Å | 2.1% |

In some embodiments, the X-ray powder diffraction (XRPD) pattern of the crystal form A of the monohydrate of the compound of formula II obtained using Cu-Kα radiation is substantially as shown in FIG. 1.

In some embodiments, the crystal form A of the monohydrate of the compound of formula II has a differential scanning calorimetry (DSC) curve showing endothermic peaks at 129.98±3°C and 196.25±3°C.

In some embodiments, the differential scanning calorimetry curve of the crystal form A of the monohydrate of the compound of formula II is as shown in FIG. 2.

In some embodiments, the crystal form A of the monohydrate of the compound of formula II has a thermogravimetric analysis (TGA) curve showing a weight loss of 2.468% within the temperature range of 30±3°C to 135±3°C.

In some embodiments, the thermogravimetric analysis curve of the crystal form A of the monohydrate of the compound of formula II is as shown in FIG. 3.

In some embodiments, the present disclosure provides a single crystal of a monohydrate of a compound of formula II, which has the following unit cell parameters: space group $P2_12_12_1$; a=6.13580(4)Å, α=90°, b=12.99957(8)Å, β=90°, c=42.4637(3)Å, γ=90°, the unit cell volume=3387.02(4)$Å^3$, the number of asymmetric units in the unit cell Z=4, the crystal density is 1.379 $mg/m^3$;

wherein the structure of the monohydrate of the compound of formula II is:

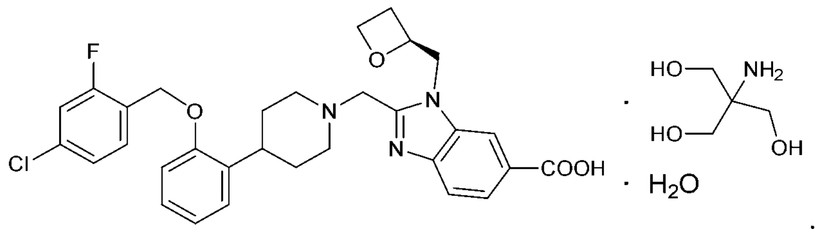

Preferably, the single crystal of the monohydrate of the compound of formula II is orthorhombic.

The present disclosure provides a crystal form B of a 1,4-dioxane solvate of a compound of formula II, which has an X-ray powder diffraction pattern obtained using Cu-Kα radiation and expressed in terms of 2θ angles, comprising diffraction peaks at 3.4±0.2°, 7.4±0.2°, 11.2±0.2°, and 14.6±0.2°;

the structure of the 1,4-dioxane solvate of the compound of formula II is:

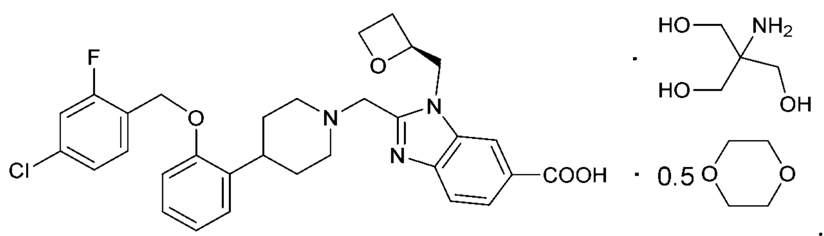

In some embodiments, the X-ray powder diffraction pattern of the crystal form B of the 1,4-dioxane solvate of the compound of formula II, obtained using Cu-Kα radiation and expressed in terms of 2θ angles, further comprises diffraction peaks at one or more of the following positions: 8.2±0.2°, 12.3±0.2°, 13.7±0.2°, 16.1±0.2°, 20.0±0.2°, 22.0±0.2°, 22.5 ±0.2°, and 29.3±0.2°.

In some embodiments, the X-ray powder diffraction pattern of the crystal form B of the 1,4-dioxane solvate of the compound of formula II, obtained using Cu-Kα radiation and expressed in terms of 2θ angles, comprises diffraction peaks at 3.4±0.2°, 7.4±0.2°, 8.2±0.2°, 11.2±0.2°, 12.3±0.2°, 13.7±0.2°, 14.6±0.2°, 16.1±0.2°, 20.0±0.2°, 22.0±0.2°, 22.5 ±0.2°, and 29.3±0.2°.

In some embodiments, the X-ray powder diffraction pattern of the crystal form B of the 1,4-dioxane solvate of the compound of formula II, obtained using Cu-Kα radiation and expressed in terms of 2θ angles, comprises diffraction peaks as shown in Table 2:

Table 2

| Number | 2θ angle | d | Relative peak intensity |
|---|---|---|---|
| 1 | 3.435° | 25.70386 Å | 5.8% |
| 2 | 7.436° | 11.87902 Å | 35.0% |
| 3 | 8.210° | 10.76082 Å | 25.6% |
| 4 | 8.923° | 9.90266 Å | 10.4% |
| 5 | 9.734° | 9.07927 Å | 6.8% |
| 6 | 11.232° | 7.87142 Å | 8.5% |
| 7 | 12.353° | 7.15933 Å | 31.7% |
| 8 | 13.769° | 6.42629 Å | 6.7% |
| 9 | 14.109° | 6.27207 Å | 11.7% |
| 10 | 14.602° | 6.06125 Å | 13.4% |
| 11 | 14.894° | 5.94310 Å | 20.1% |
| 12 | 16.133° | 5.48967 Å | 42.0% |
| 13 | 17.912° | 4.94809 Å | 11.5% |
| 14 | 20.063° | 4.42215 Å | 31.5% |
| 15 | 20.685° | 4.29067 Å | 50.3% |
| 16 | 22.002° | 4.03663 Å | 100.0% |
| 17 | 22.533° | 3.94269 Å | 50.5% |
| 18 | 23.628° | 3.76247 Å | 9.8% |
| 19 | 25.802° | 3.45007 Å | 8.8% |
| 20 | 29.387° | 3.03690 Å | 15.7% |
| 21 | 31.178° | 2.86638 Å | 9.9% |

In some embodiments, the X-ray powder diffraction (XRPD) pattern of the crystal form B of the 1,4-dioxane solvate of the compound of formula II obtained using Cu-Kα radiation is substantially as shown in FIG. 6.

In some embodiments, the crystal form B of the 1,4-dioxane solvate of the compound of formula II has a differential scanning calorimetry (DSC) curve showing endothermic peaks at 107.2±3°C and 186.6±3°C.

In some embodiments, the differential scanning calorimetry curve of the crystal form B of the 1,4-dioxane solvate of the compound of formula II is as shown in FIG. 7.

In some embodiments, the crystal form B of the 1,4-dioxane solvate of the compound of formula II has a thermogravimetric analysis (TGA) curve showing a weight loss of 2.029% within the temperature range of 33±3°C to 150 ±3°C.

In some embodiments, the thermogravimetric analysis curve of the crystal form B of the 1,4-dioxane solvate of the compound of formula II is as shown in FIG. 8.

The present disclosure provides a crystal form C of a compound of formula II, which has an X-ray powder diffraction pattern obtained using Cu-Kα radiation and expressed in terms of 2θ angles, comprising diffraction peaks at 3.7 ±0.2°, 7.5±0.2°, 9.7±0.2°, and 15.5±0.2°;

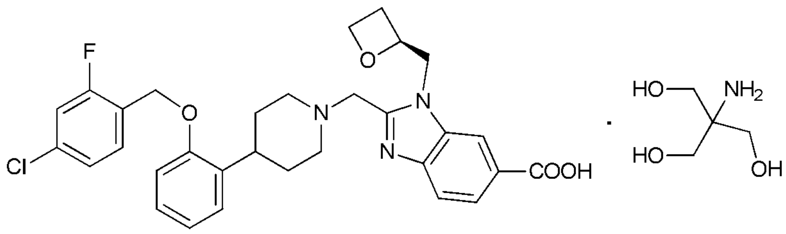

II

In some embodiments, the X-ray powder diffraction pattern of the crystal form C of the compound of formula II, obtained using Cu-Kα radiation and expressed in terms of 2θ angles, further comprises diffraction peaks at one or more of the following positions: 11.3±0.2°, 12.3±0.2°, 14.4±0.2°, 14.7±0.2°, 17.2±0.2°, 20.5±0.2°, 21.8±0.2°, and 29.5±0.2°.
In some embodiments, the X-ray powder diffraction pattern of the crystal form C of the compound of formula II, obtained using Cu-Kα radiation and expressed in terms of 2θ angles, comprises diffraction peaks at 3.7±0.2°, 7.5±0.2°, 9.7±0.2°, 11.3±0.2°, 12.3±0.2°, 14.4±0.2°, 14.7±0.2°, 15.5±0.2°, 17.2±0.2°, 20.5±0.2°, 21.8±0.2, and 29.5±0.2°.
In some embodiments, the X-ray powder diffraction pattern of the crystal form C of the compound of formula II, obtained using Cu-Kα radiation and expressed in terms of 2θ angles, comprises diffraction peaks as shown in Table 3:

Table 3

| Number | 2θ angle | d | Relative peak intensity |
|---|---|---|---|
| 1 | 3.722° | 23.71970 Å | 63.7% |
| 2 | 7.544° | 11.70969 Å | 53.0% |
| 3 | 8.211° | 10.75927 Å | 19.3% |
| 4 | 9.742° | 9.07174 Å | 100.0% |
| 5 | 11.335° | 7.80031 Å | 21.0% |
| 6 | 12.330° | 7.17280 Å | 26.5% |
| 7 | 14.468° | 6.11713 Å | 32.1% |
| 8 | 14.768° | 5.99349 Å | 55.2% |
| 9 | 15.599° | 5.67635 Å | 64.4% |
| 10 | 17.280° | 5.12770 Å | 41.6% |
| 11 | 17.708° | 5.00461 Å | 11.8% |
| 12 | 19.738° | 4.49433 Å | 42.6% |
| 13 | 20.374° | 4.35536 Å | 57.2% |
| 14 | 20.556° | 4.31726 Å | 62.7% |
| 15 | 21.880° | 4.05882 Å | 65.0% |
| 16 . | 29.519° | 3.02364 Å | 23.8% |

In some embodiments, the X-ray powder diffraction (XRPD) pattern of the crystal form C of the compound of formula II obtained using Cu-Kα radiation is substantially as shown in FIG. 9.
In some embodiments, the crystal form C of the compound of formula II has a differential scanning calorimetry (DSC) curve showing an endothermic peak at 196.9±3°C.
In some embodiments, the differential scanning calorimetry curve of the crystal form C of the compound of formula II is as shown in FIG. 10.
In some embodiments, the crystal form C of the compound of formula II has a thermogravimetric analysis (TGA) curve showing no weight loss within the temperature range of 30±3°C to 180±3°C.
In some embodiments, the thermogravimetric analysis curve of the crystal form C of the compound of formula II is as shown in FIG. 11.
In some embodiments, the crystal form C of the compound of formula II is a non-solvated crystal form.
In some embodiments, the crystal form C of the compound of formula II is free of solvent.

The present disclosure provides a crystal form D of a compound of formula II, which has an X-ray powder diffraction pattern obtained using Cu-Kα radiation and expressed in terms of 2θ angles, comprising diffraction peaks at 3.9 ±0.2°, 8.1±0.2°, 10.6±0.2°, and 14.9±0.2°;

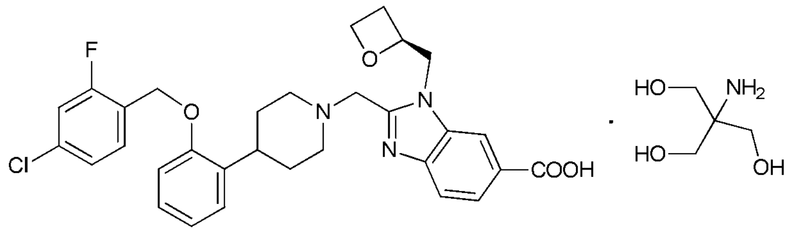

**II**

In some embodiments, the X-ray powder diffraction pattern of the crystal form D of the compound of formula II, obtained using Cu-Kα radiation and expressed in terms of 20 angles, further comprises diffraction peaks at one or more of the following positions: 10.2±0.2°, 10.6±0.2°, 14.3±0.2°, 17.8±0.2°, 20.0±0.2°, and 20.2±0.2°.

In some embodiments, the X-ray powder diffraction pattern of the crystal form D of the compound of formula II, obtained using Cu-Kα radiation and expressed in terms of 20 angles, comprises diffraction peaks at 3.9±0.2°, 8.1±0.2°, 10.2±0.2°, 10.6±0.2°, 14.3±0.2°, 14.9±0.2°, 17.8±0.2°, 20.0±0.2°, and 20.2±0.2°.

In some embodiments, the X-ray powder diffraction pattern of the crystal form D of the compound of formula II, obtained using Cu-Kα radiation and expressed in terms of 20 angles, comprises diffraction peaks as shown in Table 4:

Table 4

| Number | 2θ angle | d | Relative peak intensity |
|---|---|---|---|
| 1 | 3.959° | 22.29885 Å | 94.9% |
| 2 | 8.136° | 10.85780 Å | 86.6% |
| 3 | 10.245° | 8.62776 Å | 64.7% |
| 4 | 10.697° | 8.26398 Å | 78.4% |
| 5 | 14.334° | 6.17399 Å | 47.7% |
| 6 | 14.954° | 5.91937 Å | 81.6% |
| 7 | 15.904° | 5.56799 Å | 35.8% |
| 8 | 17.164° | 5.16195 Å | 21.4% |
| 9 | 17.898° | 4.95198 Å | 40.1% |
| 10 | 18.073° | 4.90432 Å | 35.0% |
| 11 | 20.091° | 4.41609 Å | 61.8% |
| 12 | 20.264° | 4.37868 Å | 100.0% |
| 13 | 21.600° | 4.11090 Å | 68.3% |
| 14 | 23.076° | 3.85110 Å | 15.4% |

In some embodiments, the X-ray powder diffraction (XRPD) pattern of the crystal form D of the compound of formula II obtained using Cu-Kα radiation is substantially as shown in FIG. 12.

In some embodiments, the crystal form D of the compound of formula II has a differential scanning calorimetry (DSC) curve showing endothermic peaks at 178.3±3°C and 194.0±3°C.

In some embodiments, the differential scanning calorimetry curve of the crystal form D of the compound of formula II is as shown in FIG. 13.

In some embodiments, the crystal form D of the compound of formula II has a thermogravimetric analysis (TGA) curve showing a weight loss of 0.59% within the temperature range of 30±3°C to 101±3°C, and a weight loss of 0.81% within the temperature range of 140±3°C to 180±3°C.

In some embodiments, the thermogravimetric analysis curve of the crystal form D of the compound of formula II is as shown in FIG. 14.

In some embodiments, the crystal form D of the compound of formula II is a non-solvated crystal form.

In some embodiments, the crystal form D of the compound of formula II is free of solvent.

The present disclosure provides a crystal form E of a compound of formula II, which has an X-ray powder diffraction pattern obtained using Cu-Kα radiation and expressed in terms of 2θ angles, comprising diffraction peaks at 3.7 ±0.2°, 7.6±0.2°, 9.8±0.2°, and 14.7±0.2°;

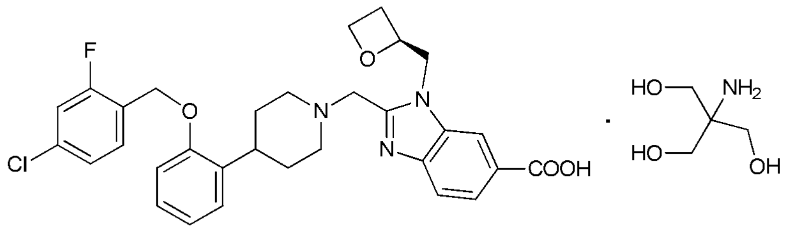

II

.

In some embodiments, the X-ray powder diffraction pattern of the crystal form E of the compound of formula II, obtained using Cu-Kα radiation and expressed in terms of 20 angles, further comprises diffraction peaks at one or more of the following positions: 11.2±0.2°, 15.6±0.2°, 17.4±0.2°, 19.8±0.2°, 20.3±0.2°, 21.9±0.2°, 29.5±0.2°, and 19.0±0.2°.

In some embodiments, the X-ray powder diffraction pattern of the crystal form E of the compound of formula II, obtained using Cu-Kα radiation and expressed in terms of 20 angles, comprises diffraction peaks at 3.7±0.2°, 7.6±0.2°, 9.8±0.2°, 11.2±0.2°, 14.7±0.2°, 15.6±0.2°, 17.4±0.2°, 19.8±0.2°, 20.3±0.2°, 21.9±0.2°, and 29.5±0.2°.

In some embodiments, the X-ray powder diffraction pattern of the crystal form E of the compound of formula II, obtained using Cu-Kα radiation and expressed in terms of 20 angles, comprises diffraction peaks as shown in Table 5:

Table 5

| Number | 2θ angle | d | Relative peak intensity |
|---|---|---|---|
| 1 | 3.725° | 23.70211 Å | 48.7% |
| 2 | 7.606° | 11.61356 Å | 44.4% |
| 3 | 9.834° | 8.98712 Å | 100.0% |
| 4 | 11.297° | 7.82653 Å | 27.8% |
| 5 | 13.430° | 6.58762 Å | 5.9% |
| 6 | 14.443° | 6.12784 Å | 32.8% |
| 7 | 14.722° | 6.01220 Å | 68.7% |
| 8 | 15.651° | 5.65749 Å | 75.3% |
| 9 | 17.416° | 5.08775 Å | 43.8% |
| 10 | 19.037° | 4.65818 Å | 9.2% |
| 11 | 19.892° | 4.45989 Å | 58.3% |
| 12 | 20.382° | 4.35373 Å | 55.8% |
| 13 | 21.980° | 4.04064 Å | 46.1% |
| 14 | 23.806° | 3.73464 Å | 7.1% |
| 15 | 26.722° | 3.33344 Å | 4.8% |
| 16 | 29.548° | 3.02073 Å | 19.7% |

In some embodiments, the X-ray powder diffraction (XRPD) pattern of the crystal form E of the compound of formula II obtained using Cu-Kα radiation is substantially as shown in FIG. 15.

In some embodiments, the crystal form E of the compound of formula II has a differential scanning calorimetry (DSC) curve showing an endothermic peak at 192.0±3°C.

In some embodiments, the differential scanning calorimetry curve of the crystal form E of the compound of formula II is as shown in FIG. 16.

In some embodiments, the crystal form E of the compound of formula II has a thermogravimetric analysis (TGA) curve showing no weight loss within the range of 30±3°C to 160±3°C.

In some embodiments, the thermogravimetric analysis curve of the crystal form E of the compound of formula II is as shown in FIG. 17.

In some embodiments, the crystal form E of the compound of formula II is a non-solvated crystal form.

In some embodiments, the crystal form E of the compound of formula II is free of solvent.

The present disclosure provides a preparation method for a crystal form of a compound of formula II, comprising the following steps:

(1) mixing the above 2-amino-2-(hydroxymethyl)-1,3-propanediol salt of the compound of formula I with a solvent to obtain a mixture;
(2) filtering the mixture and drying to obtain a crystal form of the 2-amino-2-(hydroxymethyl)-1,3-propanediol salt of the compound of formula I;

wherein when the solvent is an alcohol solvent (e.g., methanol), the crystal form A of the monohydrate of the compound of formula II is obtained;
when the solvent is 1,4-dioxane, the crystal form B of the 1,4-dioxane solvate of the compound of formula II is obtained;
when the solvent is tetrahydrofuran, the crystal form C of the compound of formula II is obtained.

In the preparation method, the mixing may be performed with stirring to form a suspension.

In the preparation method, the mixture obtained in step (1) may be a suspension.

In the preparation method, the drying is conducted in a conventional way in the art, such as drying under reduced pressure or drying under normal pressure.

In the preparation method, when the crystal form A of the monohydrate of the compound of formula II is obtained, the mass-to-volume ratio of the compound of formula II to the solvent may be 1:(1 to 100) g/mL, for example, 1:30 g/mL.

In the preparation method, when the crystal form A of the monohydrate of the compound of formula II is obtained, the temperature for mixing may be 0 to 150°C, for example, the temperature for mixing is 20 to 80°C, preferably being heated to reflux.

In the preparation method, when the crystal form A of the monohydrate of the compound of formula II is obtained, the time for mixing is conventional in the art, for example, 2 hours.

In the preparation method, when the crystal form A of the monohydrate of the compound of formula II is obtained, the filtering may be performed at room temperature, for example, filtering after cooling the mixture to room temperature.

In the preparation method, when the crystal form B of the 1,4-dioxane solvate of the compound of formula II is obtained, the mass-to-volume ratio of the compound of formula II to the solvent may be 1:(10 to 50) g/mL, for example, 1:20 g/mL.

In the preparation method, when the crystal form B of the 1,4-dioxane solvate of the compound of formula II is obtained, the temperature for mixing may be room temperature, wherein the room temperature is 20 to 30°C, for example, 25°C.

In the preparation method, when the crystal form B of the 1,4-dioxane solvate of the compound of formula II is obtained, the time for mixing is conventional in the art, for example, 24 hours.

In the preparation method, when the crystal form B of the 1,4-dioxane solvate of the compound of formula II is obtained, the mixing is conducted in a conventional way in the art, for example, mixing with stirring.

In the preparation method, when the crystal form B of the 1,4-dioxane solvate of the compound of formula II is obtained, the filtering may be performed at room temperature.

In the preparation method, when the crystal form C of the compound of formula II is obtained, the mass-to-volume ratio of the compound of formula II to the solvent may be 1:(5 to 20) g/mL, for example, 1:10 g/mL.

In the preparation method, when the crystal form C of the compound of formula II is obtained, the temperature for mixing may be 45 to 55°C, for example, 25 to 50°C, preferably 50°C.

In the preparation method, when the crystal form C of the compound of formula II is obtained, the time for mixing is conventional in the art, for example, 48 hours.

In the preparation method, when the crystal form C of the compound of formula II is obtained, the mixing is conducted in a conventional way in the art, for example, mixing with stirring.

The present disclosure provides a preparation method for the crystal form D of the compound of formula II, comprising the following steps: heating the above crystal form C of the compound of formula II or crystal form B of the 1,4-dioxane solvate of the compound of formula II to 160°C for 5 to 10 minutes to obtain (e.g., after cooling) the crystal form D of the compound of formula II.

The present disclosure provides a preparation method for the crystal form E of the compound of formula II,

comprising the following steps: heating the above crystal form B of the 1,4-dioxane solvate of the compound of formula II or crystal form C of the compound of formula II to 80 to 100°C for 24 to 96 hours (*e.g.,* 48 hours) to obtain the crystal form E of the compound of formula II.

The preparation method for the crystal form of the compound of formula II may further comprise the following steps:

step (1a): mixing the compound of formula I with solvent A to obtain mixture A;
step (1b): mixing 2-amino-2-(hydroxymethyl)-1,3-propanediol with solvent B to obtain mixture B;
steps (1a) and (1b) are in no particular order;
step (2): mixing the mixture A and the mixture B, crystallizing, filtering, and drying to obtain the compound of formula II.

In step (1a), the solvent A is a conventional solvent in the art, preferably one or more of alcohol solvents, ester solvents, ether solvents, ketone solvents, chlorinated hydrocarbon solvents, aromatic hydrocarbon solvents, nitrile solvents, alkane solvents, and nitrogen-containing compound solvents. The alcohol solvent is, for example, methanol, ethanol, or isopropanol; the ether solvent is, for example, methyl *tert*-butyl ether, isopropyl ether, tetrahydrofuran, or 2-methyltetrahydrofuran; the nitrogen-containing compound solvent is, for example, N-methylpyrrolidone; the ester solvent is, for example, methyl formate, ethyl formate, ethyl acetate, or isopropyl acetate. Further preferably, the solvent A is methanol, ethanol, acetone, ethyl acetate, a mixed solution of n-heptane and ethyl acetate, a mixed solution of *n*-heptane and tetrahydrofuran, a mixed solution of methanol and water, a mixed solution of acetone and water, a mixed solution of ethanol and water, a mixed solution of tetrahydrofuran and water, "a mixed solution of methanol, water, and dimethyl sulfoxide", "a mixed solution of 2-methyltetrahydrofuran, ethyl acetate, and methanol", "a mixed solution of ethyl acetate, tetrahydrofuran, and water" or "a mixed solution of ethyl acetate, 2-methyltetrahydrofuran, and water".

Preferably, in step (1a), the solvent A is an alcohol solvent, for example, methanol.

In step (1a), when the solvent A is "a mixed solution of 2-methyltetrahydrofuran, ethyl acetate, and methanol", the volume ratio of 2-methyltetrahydrofuran, ethyl acetate, and methanol may be 5:5:2.

In step (1a), the mass-to-volume ratio of the compound of formula I to the solution A is conventional in the art, preferably 10:(10 to 500) g/mL, for example, 10:200 g/mL.

In step (1a), the temperature for mixing is conventional in the art, for example, 10 to 160°C, preferably 20 to 30°C.

In step (1a), the mixture A may be further filtered after the mixing.

In step (1b), the solvent B is a conventional solvent in the art, preferably one or more of water, alcohol solvents, and sulfur-containing compound solvents, wherein the alcohol solvent may be ethanol or methanol, and the sulfur-containing compound solvent may be dimethyl sulfoxide.

Preferably, in step (1b), the solvent B is water.

In step (1b), the mass ratio of 2-amino-2-(hydroxymethyl)-1,3-propanediol to the solvent B is conventional in the art, for example, 1:(1 to 260), preferably 3.2:800.

In step (2), the mixing is conducted in a conventional way in the art, for example, the mixing is performed by adding the mixture B dropwise to the mixture A.

In step (2), the temperature for crystallizing is conventional in the art, for example, 20 to 30°C.

In step (2), the time for crystallizing is under conventional conditions in the art, generally it will be done until the compound of formula II no longer precipitates. For example, the time for mixing is 1 to 24 hours, and for a preferred example, 12 to 24 hours.

In step (2), the filtering may comprise steps of filtering, washing (e.g., washing with the solution A and/or the solution B), and pH adjustment (e.g., washing with water to pH=7 to 8).

In step (2), the drying is conducted in a conventional way in the art, such as drying under vacuum at 45°C.

The present disclosure provides a pharmaceutical composition comprising the above 2-amino-2-(hydroxymethyl)-1,3-propanediol salt of the compound of formula I or the solvate thereof (the solvate of the 2-amino-2-(hydroxymethyl)-1,3-propanediol salt of the compound of formula I), and a pharmaceutically acceptable carrier;
preferably, the 2-amino-2-(hydroxymethyl)-1,3-propanediol salt of the compound of formula I or the solvate thereof is the above single crystal of the monohydrate of the compound of formula II, crystal form A of the monohydrate of the compound of formula II, crystal form B of the 1,4-dioxane solvate of the compound of formula II, crystal form C of the compound of formula II, crystal form D of the compound of formula II, or crystal form E of the compound of formula II.

The present disclosure provides a use of the above pharmaceutical composition or the above 2-amino-2-(hydroxymethyl)-1,3-propanediol salt of the compound of formula I or the solvate thereof (the solvate of the 2-amino-2-(hydroxymethyl)-1,3-propanediol salt of the compound of formula I) in the manufacture of a medicament for preventing and/or treating metabolism-related diseases;
preferably, the 2-amino-2-(hydroxymethyl)-1,3-propanediol salt of the compound of formula I or the solvate thereof is the above single crystal of the monohydrate of the compound of formula II, crystal form A of the monohydrate of the compound of formula II, crystal form B of the 1,4-dioxane solvate of the compound of formula II, crystal form C of the compound of

formula II, crystal form D of the compound of formula II, or crystal form E of the compound of formula II.

In the use, the metabolism-related diseases may be selected from any one of glucose intolerance, hyperglycemia, dyslipidemia, type 1 diabetes (T1DM), type 2 diabetes (T2DM), hypertriglyceridemia, insulin resistance, impaired glucose tolerance (IGT), diabetic dyslipidemia, hyperlipidemia, arteriosclerosis, atherosclerosis, hypertension, obesity, non-alcoholic fatty liver disease, non-alcoholic steatohepatitis, liver fibrosis, cirrhosis, and lethargy.

The above preferred conditions can be combined arbitrarily to obtain preferred embodiments of the present disclosure without violating common knowledge in the art.

The reagents and starting materials used in the present disclosure are all commercially available.

The positive and progressive effects of the present disclosure are that the GLP-1R receptor agonist compound salts provided by the present disclosure have good stability and excellent solid properties. They are stable under compressing, grinding, and high humidity conditions. Therefore, they have good medicinal prospects.

BRIEF DESCRIPTION OF THE DRAWINGS

FIG. 1 is an X-ray powder diffraction pattern of the crystal form A of the monohydrate of the compound of formula II.
FIG. 2 is a differential scanning calorimetry curve of the crystal form A of the monohydrate of the compound of formula II.
FIG. 3 is a thermogravimetric analysis curve of the crystal form A of the monohydrate of the compound of formula II.
FIG. 4 is a single crystal simulated X-ray powder diffraction pattern of the monohydrate of the compound of formula II.
FIG. 5 is a single crystal structure of the monohydrate of the compound of formula II.
FIG. 6 is an X-ray powder diffraction pattern of the crystal form B of the 1,4-dioxane solvate of the compound of formula II.
FIG. 7 is a differential scanning calorimetry curve of the crystal form B of the 1,4-dioxane solvate of the compound of formula II.
FIG. 8 is a thermogravimetric analysis curve of the crystal form B of the 1,4-dioxane solvate of the compound of formula II.
FIG. 9 is an X-ray powder diffraction pattern of the crystal form C of the compound of formula II.
FIG. 10 is a differential scanning calorimetry curve of the crystal form C of the compound of formula II.
FIG. 11 is a thermogravimetric analysis curve of the crystal form C of the compound of formula II.
FIG. 12 is an X-ray powder diffraction pattern of the crystal form D of the compound of formula II.
FIG. 13 is a differential scanning calorimetry curve of the crystal form D of the compound of formula II.
FIG. 14 is a thermogravimetric analysis curve of the crystal form D of the compound of formula II.
FIG. 15 is an X-ray powder diffraction pattern of the crystal form E of the compound of formula II.
FIG. 16 is a differential scanning calorimetry curve of the crystal form E of the compound of formula II.
FIG. 17 is a thermogravimetric analysis curve of the crystal form E of the compound of formula II.
FIG. 18 is an X-ray powder diffraction pattern of the crystal form I of the compound of formula I.
FIG. 19 is an X-ray powder diffraction pattern of the crystal form II of the compound of formula I.
FIG. 20 is an X-ray powder diffraction pattern of the crystal form III of the compound of formula I.
FIG. 21 is an X-ray powder diffraction pattern of the tableting and grinding test for the crystal form I of the compound of formula I.
FIG. 22 is an X-ray powder diffraction pattern of the tableting and grinding test for the crystal form II of the compound of formula I.
FIG. 23 is an X-ray powder diffraction pattern of the tableting and grinding test for the crystal form III of the compound of formula I.
FIG. 24 is an X-ray powder diffraction pattern of the tableting test for the crystal form A of the monohydrate of the compound of formula II.
FIG. 25 is an X-ray powder diffraction pattern of the tableting and grinding test for the crystal form A of the monohydrate of the compound of formula II.

DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples should be selected according to conventional methods and conditions, or according to product specifications.

The actual and adopted methods used in the examples of the present disclosure are all conventional reagents and conventional methods in the art. It should be clear to those skilled in the art that, hereinafter, unless otherwise

specified, the temperature is expressed in degrees Celsius (°C). The operating temperature is carried out at room temperature, where the room temperature ranges from 10 to 30°C.

Instruments and methods:

The obtained reaction samples were analyzed using a variety of detection and analysis methods, wherein (1) X-ray diffraction patterns were collected using a Bruker D2 Phaser instrument with the following instrument parameters:

| | | Detection mode | Step measurement |
|---|---|---|---|
| X-ray emitter | Cu, k-Alphal ($\lambda$=1.54184Å) | Tube voltage (kV) | 30 |
| Tube current (mA) | 10 | Divergence slit (mm) | 0.6 |
| Primary optical path axial Soller slit (°) | 2.5 | Secondary optical path axial Soller slit (°) | 2.5 |
| Detector slit (°) | 5.827 | Anti-scatter slit (mm) | 0 |
| Scan axis | $\theta$s- $\theta$d | Step size (deg) | 0.02 |
| Dwell time per step (S) | 0.2 | Scanning range (deg) | 3 to 40 |

(2) DSC
The DSC curve was acquired using a TA Instrument DSC 250. The sample was weighed into a sample pan, accurately weighed, and the weight was recorded. The sample was then tested after puncturing in an aluminum crucible. The temperature was increased from 25°C to a final temperature of 300°C at a heating rate of 10°C/min.
(3) TGA
The TGA curve was acquired using a TA Instrument TGA 550. The sample was weighed into a sample pan and subjected to an open air test, with a heating rate of 10°C/min from 40°C to 300°C.
(4) HPLC, measurement parameters are shown in the following table:

| | |
|---|---|
| Instrument | Agilent 1290 Infinity |
| Chromatographic column | AGILENT ECLIPSE XDB-C18 Column 4.6×150 mm, 5 µm PN: 993967-902 |
| Injection volume | 0.3 µL |
| Detection wavelength | 220 nm |
| Injection concentration | 2.0 mg/mL |
| Mobile phase | A: 0.1% HCOOH-aqueous solution<br>B: 0.1% HCOOH-acetonitrile solution |

| | |
|---|---|
| Gradient (T/B%) | <table><tr><td>Time (min)</td><td>B%</td></tr><tr><td>0.00</td><td>10</td></tr><tr><td>5</td><td>100</td></tr><tr><td>15</td><td>100</td></tr></table> |
| Column temperature | 25°C |
| Run time | 15 min |
| Flow rate | 1.0 mL/min |
| Diluent | Methanol |

Detection method:

Purity test: The sample to be tested was dissolved in a methanol solution, and the sample purity was measured by HPLC.

Hygroscopicity test method (DVS): The hygroscopicity of the sample was measured using SMS Intrinsic Plus. The sample was weighed into a sample pan, equilibrated at 25°C until dm/dt was less than 0.002%, and then measured in time mode. The humidity was adjusted from 60%-90%-0%-90%-0% RH, with increments of 10% RH per step, and each humidity level was maintained for 1 hour.

Grindability test: An appropriate amount of the crystal compound to be tested was placed in a mortar and ground for 2 min and 5 min, followed by XRPD detection.

Compressibility test: The crystal compounds to be tested were compressed into tablets under 20 mPa pressure, and the XRPD results were subsequently detected.

Solubility test:

Approximately 10 mg of the crystal compound was weighed and added to 3 mL of water, shaken, and maintained at 37°C. After 24 hours, a sample was taken, and its solubility was determined by HPLC.

**Example 1:** Hydrochloride salt of (S)-2-((4-(2-((4-chloro-2-fluorobenzyl)oxy)phenyl)piperidin-1-yl)methyl)-1-(oxetan-2-aminomethyl)-1H-benzo[d]imidazole-6-carboxylate

Methanol (20 mL) was added to a flask, followed by the dropwise addition of acetyl chloride (1.53 g). After the dropwise addition was complete, the mixture was stirred for 1 hour and set aside.

(*S*)-2-((4-(2-((4-Chloro-2-fluorobenzyl)oxy)phenyl)piperidin-1-yl)methyl)-1-(oxetan-2-aminomethyl)-1*H*-benzo[*d*]imidazole-6-carboxylic acid (10 g) *(i.e.,* the compound of formula I, prepared according to the method described in Example 3 of patent CN202110334388) was added to methanol (200 mL), and the mixture was stirred at 20 to 30°C. The above methanol solution of acetyl chloride was slowly added dropwise to the methanol solution of the compound of formula I. After the addition was complete, the mixture was stirred for 1 hour, followed by the dropwise addition of water (100 mL). After stirring for 3 hours, the mixture was filtered, and the filter cake was washed twice with methanol/water (50 mL×2, 2:1). The filter cake was dried under vacuum at 45°C to constant weight to obtain 9.1 g of the product. $^1$H NMR (400MHz $CD_3OD$) 9.62-9.72 (1H, m), 8.12 (1H, d), 7.99-8.01 (1H, dd), 7.72-7.73 (1H, d), 7.45-7.48 (1H, m), 7.32-7.34 (1H, dd), 7.22-7.26 (3H, m), 7.09-7.12 (1H, td), 6.85-6.86 (1H, dd), 5.19-5.20 (2H, d), 4.55-4.60 (1H, q), 4.09-4.22 (2H, ABq), 3.94-3.96 (1H, d), 3.77-3.89 (2H, m), 3.06-3.20 (4H, m), 2.31-2.48 (5H, m), 2.22-2.29 (1H, m).

HPLC purity: 94.74%.

**Example 2:** Sodium salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid (2 g) (prepared according to the method described in Example 3 of patent CN202110334388) was added to dimethyl sulfoxide (1 mL), and the mixture was stirred at 20 to 30°C. A sodium hydroxide solution (3.75 mL, 1 mol/L) was added dropwise, followed by the dropwise addition of acetone (20 mL) under stirring. After stirring for 5 hours, the mixture was subjected to suction filtration. The filter cake was quickly washed twice with acetone and then transferred to a flask for drying under reduced pressure to obtain an off-white solid (1.5 g). $^1$H NMR (400MHz $CD_3OD$) 8.13 (1H, m), 8.09-8.10 (1H, dd), 7.74-7.76 (1H, d), 7.41-7.44 (1H,ddt), 7.31-7.32(1H, dd), 7.23-7.26 (3H, m), 7.15-7.11 (1H, m), 6.85-6.87 (1H, m), 5.19-5.20 (2H, d), 4.55-4.60 (1H, m), 4.04-4.21 (2H, ABq), 3.77-3.88 (2H, m), 3.63 (2H, s), 2.85-2.95 (3H, m), 2.72-2.78 (2H, m). HPLC purity: 99.90%.

**Example 3:** 2-Amino-2-(hydroxymethyl)-1,3-propanediol salt of (S)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid (10 g, prepared according to the method described in Example 3 of patent CN202110334388) was added to methanol (200 mL), and the mixture was stirred at 20 to 30°C until clear, followed by filtration. Tris(hydroxymethyl)aminomethane (3.2 g) was dissolved in water (800 g) and slowly added dropwise to the above reaction mixture under stirring at 20 to 30°C, and a solid precipitated. The mixture was stirred for 12 to 24 hours. After filtration, the filter cake was washed twice with methanol/water (500 mL×2, 2:1) and then washed with water until pH=7-8. The filter cake was dried under vacuum at 45°C to constant weight to obtain 11.0 g of the product, *i.e.,* the compound of formula II. $^1$H NMR (400MHz $CD_3OD$) 8.21 (1H, d), 7.96-7.97 (1H, dd), 7.94-7.95 (1H, d), 7.50-7.61 (1H, td), 7.25-7.29 (2H, m), 7.20-7.22 (2H, m), 6.96-7.02 (1H, dd), 6.94-6.96 (1H, t), 5.12 (1H, m), 4.95 (2H, s), 4.85-4.89 (2H, m), 4.73-4.74 (1H, m), 4.63-4.70 (1H, m), 4.47-4.48 (1H, m), 3.67-4.46 (2H, ABq), 3.67 (6H, s), 2.96-3.02 (3H, m), 2.78-2.88 (1H, m), 2.48-2.58 (1H, m)2.20-2.28 (2H, m)1.75-1.80 (4H, m). HPLC purity: 99.95%.

**Example 3-1:** 2-Amino-2-(hydroxymethyl)-1,3-propanediol salt of (S)-2-((4-(2-((4-chloro-2-fluorobenzyl)oxy)phenyl)piperidin-1-yl)methyl)-1-(oxetan-2-aminomethyl)-1*H*-benzo[d]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid (10 g, prepared according to the method described in Example 3 of patent CN202110334388) was added to tetrahydrofuran (400 mL), and the mixture was stirred at 20 to 30°C until clear, followed by filtration. Tris(hydroxymethyl)aminomethane (2.1 g) was dissolved in water (150 g) and slowly added dropwise to the above reaction mixture under stirring at 20 to 30°C, and a solid precipitated. The mixture was stirred for 10 to 16 hours. After filtration, the filter cake was washed twice with tetrahydrofuran/water (80 mL×2, 3:1) and then washed with water until pH=7-8. The filter cake was dried under vacuum at 45°C to constant weight to obtain 11.4 g of the product, *i.e.,* the compound of formula II.

**Example 3-2:** 2-Amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid (10 g, prepared according to the method described in Example 3 of patent CN202110334388) was added to *N*-methylpyrrolidone (20 mL), and the mixture was stirred at 120 to 150°C until clear, followed by filtration. Tris(hydroxymethyl)aminomethane (4.2 g) was dissolved in water (50 g) and slowly added dropwise to the above reaction mixture under stirring at 20 to 30°C, and a solid precipitated. The mixture was stirred for 10 to 16 hours. After filtration, the filter cake was washed twice with ethyl acetate (30 mL×2) and then washed with water until pH=7-8. The filter cake was dried under vacuum at 45°C to constant weight to obtain 9.1 g of the product, *i.e.,* the compound of formula II.

**Example 3-3:** 2-Amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo [*d*]imidazole-6-carboxylic acid (10 g, prepared according to the method described in Example 3 of patent CN202110334388) was added to 2-methyltetrahydrofuran (50 mL), ethyl acetate (50 mL), and methanol (20 mL), and

the mixture was stirred at 20 to 30°C until clear, followed by filtration. Tris(hydroxymethyl)aminomethane (2.1 g) was dissolved in water (30 g) and slowly added dropwise to the above reaction mixture under stirring at 20 to 30°C, and a solid precipitated. The mixture was stirred for 2 to 3 hours. After filtration, the filter cake was washed twice with ethyl acetate (10 mL×2) and then washed with water until pH=7-8. The filter cake was dried under vacuum at 45°C to constant weight to obtain 11.9 g of the product, *i.e.,* the compound of formula II.

**Example 3-4:** 2-Amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate

(*S*)-2-((4-(2-((4-Chloro-2-fluorobenzyl)oxy)phenyl)piperidin-1-yl)methyl)-1-(oxetan-2-aminomethyl)-1*H*-benzo[*d*]imidazole-6-carboxylic acid (10 g, prepared according to the method described in Example 3 of patent CN202110334388) was added to ethanol (300 mL), and the mixture was stirred at 78°C until clear, followed by filtration. Tris(hydroxymethyl)aminomethane (3.2 g) was dissolved in ethanol (100 mL) and slowly added dropwise to the above reaction mixture under stirring at 20 to 30°C, and a solid precipitated. The mixture was stirred for about 8 hours. After filtration, the filter cake was washed twice with ethanol (50 mL×2). The filter cake was dried under vacuum at 45°C to constant weight to obtain 12.0 g of the product, *i.e.,* the compound of formula II.

Crystal transformation process

**Example 4:** Crystal form A of the monohydrate of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (1 g) prepared in Example 3 was added to methanol (30 mL), stirred, heated to reflux for 2 hours, naturally cooled to room temperature, stirred for an additional 2 hours, filtered to obtain a solid, and dried under reduced pressure to constant weight to obtain 0.80 g of the product, which is the crystal form A of the monohydrate of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II).

The XRPD pattern analysis data of the crystal form A of the monohydrate of the compound of formula II are shown in Table 1.

Table 1

| Number | 2θ angle | d | Relative peak intensity |
|---|---|---|---|
| 1 | 4.114° | 21.46041 Å | 19.8% |
| 2 | 8.295° | 10.65090 Å | 91.1% |
| 3 | 10.703° | 8.25895 Å | 80.6% |
| 4 | 12.388° | 7.13911 Å | 100.0% |
| 5 | 14.151° | 6.25379 Å | 64.9% |
| 6 | 14.941° | 5.92472 Å | 65.2% |
| 7 | 15.954° | 5.55077 Å | 31.3% |
| 8 | 16.566° | 5.34683 Å | 13.9% |
| 9 | 17.086° | 5.18547 Å | 16.0% |
| 10 | 17.948° | 4.93836 Å | 18.9% |
| 11 | 18.429° | 4.81041 Å | 17.4% |
| 12 | 19.019° | 4.66256 Å | 36.0% |
| 13 | 19.916° | 4.45440 Å | 19.7% |
| 14 | 20.248° | 4.38230 Å | 21.0% |
| 15 | 20.530° | 4.32259 Å | 48.4% |
| 16 | 20.768° | 4.27354 Å | 94.2% |

(continued)

| Number | 2θ angle | d | Relative peak intensity |
|---|---|---|---|
| 17 | 21.362° | 4.15615 Å | 40.2% |
| 18 | 21.868° | 4.06113 Å | 70.7% |
| 19 | 22.405° | 3.96502 Å | 28.4% |
| 20 | 23.059° | 3.85389 Å | 18.2% |
| 21 | 23.528° | 3.77811 Å | 12.6% |
| 22 | 23.874° | 3.72426 Å | 22.6% |
| 23 | 24.612° | 3.61423 Å | 5.5% |
| 24 | 25.081° | 3.54767 Å | 23.8% |
| 25 | 25.383° | 3.50611 Å | 14.5% |
| 26 | 25.899° | 3.43736 Å | 42.5% |
| 27 | 26.264° | 3.39049 Å | 30.7% |
| 28 | 26.667° | 3.34010 Å | 18.2% |
| 29 | 27.176° | 3.27878 Å | 4.8% |
| 30 | 27.646° | 3.22405 Å | 11.1% |
| 31 | 27.968° | 3.18764 Å | 4.4% |
| 32 | 28.847° | 3.09251 Å | 7.2% |
| 33 | 29.734° | 3.00219 Å | 20.1% |
| 34 | 30.268° | 2.95045 Å | 2.4% |
| 35 | 30.988° | 2.88351 Å | 8.4% |
| 36 | 31.535° | 2.83476 Å | 8.1% |
| 37 | 32.098° | 2.78633 Å | 11.5% |
| 38 | 32.770° | 2.73066 Å | 2.7% |
| 39 | 33.246° | 2.69269 Å | 1.9% |
| 40 | 34.317° | 2.61104 Å | 9.7% |
| 41 | 34.659° | 2.58607 Å | 4.3% |
| 42 | 35.356° | 2.53667 Å | 9.7% |
| 43 | 36.387° | 2.46713 Å | 2.7% |
| 44 | 37.305° | 2.40846 Å | 4.9% |
| 45 | 37.817° | 2.37704 Å | 9.3% |
| 46 | 38.537° | 2.33425 Å | 2.8% |
| 47 | 39.257° | 2.29310 Å | 2.1% |

.

The characterization data of the crystal form A of the monohydrate of the compound of formula II are as follows:

| Parameters | Method | Result |
|---|---|---|
| X-ray diffraction | 3 to 40° (2θ) | High crystallinity, see FIG. 1; |

(continued)

| Parameters | Method | Result |
|---|---|---|
| Thermal analysis | DSC, 10°C/min | endothermic peaks at 129.98±3°C (onset temperature: 102.10°C, enthalpy (normalized): 79.264±0.2 J/g), 196.25±3°C (onset temperature: 192.33°C, enthalpy: 63.349 ±0.2 J/g), and 197.44±3°C (onset temperature: 173.24°C, enthalpy: 6.2628±0.2 J/g), see FIG. 2; exothermic peaks shown in the upward direction; |
| Thermal weight loss | TGA, 10°C/min | approximately 2.468% weight loss at 30 to 135°C, see FIG. 3. |

The obtained crystal form A of the monohydrate of the compound of formula II is a single crystal of the monohydrate of the compound of formula II, with its simulated XRPD pattern shown in FIG. 4, its single crystal structure shown in FIG. 5, and its main crystal parameter data as follows:

| | |
|---|---|
| Unit cell dimensions $mm^3$ | 0.30×0.10×0.04 $mm^3$ |
| Target type | CuKa (λ=1.54184) |
| Crystal system | Orthorhombic |
| Space group | $P2_12_12_1$ |
| a/Å | 6.13580(4) |
| b/Å | 12.99957(8) |
| c/Å | 42.4637(3) |
| α/° | 90 |
| β/° | 90 |
| γ/° | 90 |
| Unit cell volume/$Å^3$ | 3387.02(4) |
| Number of molecules per unit cell | 4 |
| Unit cell density $g/cm^3$ | 1.379 |
| F(000) | 1488.0 |
| Absorption-related factor $\mu/mm^{-1}$ | 1.540 |
| Diffraction index range (h/k/l) | -7≤h≤7, -15≤k≤15, -39≤1≤50 |
| Unit cell measurement temperature/K | 149.99(10) |
| Data collection range/° | 7.112 to 133.2 |
| Structure refinement factor $F^2$ | 1.042 |
| Residual factor R for observable diffraction points ⌈I>=2σ(I)⌋ | $R_1$=0.0239, $wR_2$=0.0629 |
| Residual factor R for observable diffraction points [all data] | $R_1$=0.0243, $wR_2$=0.0632 |
| Peak/valley of residual electron density after refinement/$eÅ^{-3}$ | 0.16/-0.17 |
| Diffraction point collection/unique | 60146/5986[Rint=0.0502] |
| Absolute configuration parameter . | 0.004(5) |

**Example 5:** Crystal form B of the 1,4-dioxane solvate of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (2 g) prepared in Example 3 was

added to 1,4-dioxane (40 mL), stirred at room temperature for 24 hours, filtered to obtain a solid, and dried under reduced pressure to constant weight to obtain 1.90 g of the product *(i.e.,* the crystal form B of the 1,4-dioxane solvate of the compound of formula II, wherein the molar ratio of the compound of formula II to 1,4-dioxane is 1:0.5). $^1$H NMR (400MHz $CD_3OD$) 8.22 (1H, s), 7.95-7.97 (1H, dd), 7.60-7.62 (1H, d), 7.51-7.53 (1H, t), 7.27-2.29 (2H, m), 7.15-7.21 (1H, m), 7.01-7.05 (1H, d), 6.93-6.97 (1H, t), 5.27-5.30 (1H, m), 4.63-4.75 (2H, m), 4.47-4.49 (1H, m), 3.85-4.03 (2H, ABq), 3.67-3.68 (10H, m), 3.32-3.34 (4H, m), 2.94-3.06 (3H, m), 2.79-2.83 (1H, m), 2.52-2.57 (1H, m), 2.22-2.34 (1H, m), 1.73-1.82 (4H, m). HPLC purity: 99.99%.

The XRPD pattern analysis data for the crystal form B of the 1,4-dioxane solvate of the compound of formula II are shown in Table 2.

Table 2

| Number | 2θ angle | d | Relative peak intensity |
|---|---|---|---|
| 1 | 3.435° | 25.70386 Å | 5.8% |
| 2 | 7.436° | 11.87902 Å | 35.0% |
| 3 | 8.210° | 10.76082 Å | 25.6% |
| 4 | 8.923° | 9.90266 Å | 10.4% |
| 5 | 9.734° | 9.07927 Å | 6.8% |
| 6 | 11.232° | 7.87142 Å | 8.5% |
| 7 | 12.353° | 7.15933 Å | 31.7% |
| 8 | 13.769° | 6.42629 Å | 6.7% |
| 9 | 14.109° | 6.27207 Å | 11.7% |
| 10 | 14.602° | 6.06125 Å | 13.4% |
| 11 | 14.894° | 5.94310 Å | 20.1% |
| 12 | 16.133° | 5.48967 Å | 42.0% |
| 13 | 17.912° | 4.94809 Å | 11.5% |
| 14 | 20.063° | 4.42215 Å | 31.5% |
| 15 | 20.685° | 4.29067 Å | 50.3% |
| 16 | 22.002° | 4.03663 Å | 100.0% |
| 17 | 22.533° | 3.94269 Å | 50.5% |
| 18 | 23.628° | 3.76247 Å | 9.8% |
| 19 | 25.802° | 3.45007 Å | 8.8% |
| 20 | 29.387° | 3.03690 Å | 15.7% |
| 21 | 31.178° | 2.86638 Å | 9.9% |

The characterization data of the crystal form B of the 1,4-dioxane solvate of the compound of formula II are shown in the following table:

| Parameters | Method | Result |
|---|---|---|
| X-ray diffraction | 3 to 40° (2θ) | Low crystallinity, see FIG. 6; |
| Thermal analysis | DSC, 10°C/min | endothermic peaks at 107.23±3°C (onset temperature: 91.64°C, enthalpy (normalized): 4.2937±0.2 J/g) and 186.64±3°C (onset temperature: 169.74°C, enthalpy (normalized): 1.5641±0.2 J/g), see FIG. 7; exothermic peaks shown in the upward direction; |
| Thermal weight loss | TGA, 10°C/min | approximately 2.029% weight loss at 33 to 150°C, see FIG. 8. |

**Example 6:** Crystal form C of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (2 g) prepared in Example 3 was added to tetrahydrofuran (20 mL), stirred at 25 to 50°C for 48 hours, filtered to obtain a solid, and dried under reduced pressure to constant weight to obtain 2.10 g of the product (crystal form C of the compound of formula II). $^1H$ NMR (400MHz DMSO-$d_6$) 8.19 (1H, d), 7.79-7.81 (1H, dd), 7.51-7.60 (2H, m), 7.36-7.39 (1H, dd), 7.16-7.21 (2H, m), 7.07-7.09 (1H, dd), 6.92-6.96 (1H, td), 5.15 (2H, s), 5.06-5.15 (1H, m), 4.73-4.79 (2H, dd), 4.60-4.65 (1H, dd), 4.47-4.52 (1H, m), 4.34-4.39 (1H, m), 3.75-3.96 (2H, ABq), 3.55-3.61 (m), 3.38 (6H, s), 2.96-2.99 (1H, d), 2.85-2.90 (2H, m), 2.66-2.75 (1H, m), 2.38-2.45 (2H, m), 2.10-2.22 (2H, m), 1.54-1.71 (4H, m). The XRPD pattern analysis data of the crystal form C of the compound of formula II are shown in Table 3.

Table 3

| Number | 2θ angle | d | Relative peak intensity |
|---|---|---|---|
| 1 | 3.722° | 23.71970 Å | 63.7% |
| 2 | 7.544° | 11.70969 Å | 53.0% |
| 3 | 8.211° | 10.75927 Å | 19.3% |
| 4 | 9.742° | 9.07174 Å | 100.0% |
| 5 | 11.335° | 7.80031 Å | 21.0% |
| 6 | 12.330° | 7.17280 Å | 26.5% |
| 7 | 14.468° | 6.11713 Å | 32.1% |
| 8 | 14.768° | 5.99349 Å | 55.2% |
| 9 | 15.599° | 5.67635 Å | 64.4% |
| 10 | 17.280° | 5.12770 Å | 41.6% |
| 11 | 17.708° | 5.00461 Å | 11.8% |
| 12 | 19.738° | 4.49433 Å | 42.6% |
| 13 | 20.374° | 4.35536 Å | 57.2% |
| 14 | 20.556° | 4.31726 Å | 62.7% |
| 15 | 21.880° | 4.05882 Å | 65.0% |
| 16 . | 29.519° | 3.02364 Å | 23.8% |

The characterization data of the crystal form C of the compound of formula II are as follows:

| Parameters | Method | Result |
|---|---|---|
| X-ray diffraction | 3 to 40° (2θ) | Moderate crystallinity, see FIG. 9; |
| Thermal analysis | DSC, 10°C/min | endothermic peaks at 196.97±3°C (onset temperature: 190.27°C, enthalpy (normalized): 72.307±0.2 J/g) and 169.36±3°C (onset temperature: 162.20°C, enthalpy (normalized): 6.6069±0.2 J/g), see FIG. 10; exothermic peaks shown in the upward direction; |
| Thermal weight loss | TGA, 10°C/min | No weight loss at 30 to 180°C, see FIG. 11; |

**Example 7:** Crystal form D of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1H-benzo[d]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (0.50 g, crystal form B of the 1,4-dioxane solvate of the compound of formula II or crystal form C of the compound of formula II) prepared in Example 5 or 6 was heated to 160°C, maintained for 5 to 10 min, and cooled to obtain 0.46 g of a solid *(i.e.,* crystal form D of the compound of formula II). $^1$H NMR (400 MHz, $CD_3OD$) δ 8.22 (1H, s), 7.95-7.97 (1H, dd), 7.60-7.62 (1H, d), 7.52-7.56 (1H, t), 7.27-7.30 (2H, m), 7.21-7.24 (1H, dd), 7.15-7.20 (1H, td), 7.02-7.04 (1H, dd), 6.93-6.97 (1H, td), 5.25-5.32 (1H, qd), 5.14 (1H, s), 4.71-4.75 (1H, dd), 4.62-4.67 (1H, m), 4.45-4.50 (1H, m), 3.90-4.03 (2H, ABq), 3.66 (6H, s), 2.95-3.06 (4H, m), 2.77-2.85 (1H, m), 2.50-2.59 (1H, m), 2.22-2.34 (1H, m), 1.71-1.81 (4H, m). HPLC purity: 99.9%.

The XRPD pattern analysis data of the crystal form D of the compound of formula II are shown in Table 4.

Table 4

| Number | 2θ angle | d | Relative peak intensity |
|---|---|---|---|
| 1 | 3.959° | 22.29885 Å | 94.9% |
| 2 | 8.136° | 10.85780 Å | 86.6% |
| 3 | 10.245° | 8.62776 Å | 64.7% |
| 4 | 10.697° | 8.26398 Å | 78.4% |
| 5 | 14.334° | 6.17399 Å | 47.7% |
| 6 | 14.954° | 5.91937 Å | 81.6% |
| 7 | 15.904° | 5.56799 Å | 35.8% |
| 8 | 17.164° | 5.16195 Å | 21.4% |
| 9 | 17.898° | 4.95198 Å | 40.1% |
| 10 | 18.073° | 4.90432 Å | 35.0% |
| 11 | 20.091° | 4.41609 Å | 61.8% |
| 12 | 20.264° | 4.37868 Å | 100.0% |
| 13 | 21.600° | 4.11090 Å | 68.3% |
| 14 | 23.076° | 3.85110 Å | 15.4% |

The characterization data of the crystal form D of the compound of formula II are as follows:

| Parameters | Method | Result |
|---|---|---|
| X-ray diffraction | 3 to 40° (2θ) | Low crystallinity, see FIG. 12; |
| Thermal analysis | DSC, 10°C/min | endothermic peaks at 178.35±3°C (onset temperature: 174.06°C, enthalpy (normalized): 8.2915±0.2 J/g) and 194.04±3°C (onset temperature: 185.80°C, enthalpy: 42.972±0.2 J/g), see FIG. 13;<br>exothermic peaks shown in the upward direction; |
| Thermal weight | TGA, 10°C/min | weight loss of 0.597% at 30 to 101°C and |
| loss | | weight loss of 0.812% at 140 to 181°C, see FIG. 14. |

**Example 8:** Crystal form E of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (0.20 g, crystal form B of the 1,4-dioxane solvate of the compound of formula II or crystal form C of the compound of formula II) prepared in Example 5 or 6 was heated to 80 to 100°C and dried for 48 hours to obtain 0.18 g of a solid *(i.e.,* crystal form E of the compound of formula II). $^1$H NMR (400 MHz, $CD_3OD$) δ 8.22 (1H, s), 7.95-7.97 (1H, dd), 7.60-7.62 (1H, d), 7.51-7.55 (1H, t), 7.27-7.30 (2H, m), 7.21-7.23 (1H, m), 7.15-7.20 (1H, td), 7.01-7.04 (1H, dd), 6.93-6.97 (1H, td), 5.25-5.32 (1H, qd), 5.14 (1H, s), 4.71-4.75 (1H, dd), 4.62-4.67 (1H, m), 4.45-4.50 (1H, m), 3.90-4.03 (2H, ABq), 3.64 (6H, s), 2.94-3.06 (4H, m), 2.77-2.85 (1H, m), 2.50-2.59 (1H, m), 2.22-2.34 (1H, m), 1.75-1.81 (4H, m). HPLC purity: 99.9%

The XRPD pattern analysis data of the crystal form E of the compound of formula II are shown in Table 5.

Table 5

| Number | 2θ angle | d | Relative peak intensity |
|---|---|---|---|
| 1 | 3.725° | 23.70211 Å | 48.7% |
| 2 | 7.606° | 11.61356 Å | 44.4% |
| 3 | 9.834° | 8.98712 Å | 100.0% |
| 4 | 11.297° | 7.82653 Å | 27.8% |
| 5 | 13.430° | 6.58762 Å | 5.9% |
| 6 | 14.443° | 6.12784 Å | 32.8% |
| 7 | 14.722° | 6.01220 Å | 68.7% |
| 8 | 15.651° | 5.65749 Å | 75.3% |
| 9 | 17.416° | 5.08775 Å | 43.8% |
| 10 | 19.037° | 4.65818 Å | 9.2% |
| 11 | 19.892° | 4.45989 Å | 58.3% |
| 12 | 20.382° | 4.35373 Å | 55.8% |
| 13 | 21.980° | 4.04064 Å | 46.1% |
| 14 | 23.806° | 3.73464 Å | 7.1% |
| 15 | 26.722° | 3.33344 Å | 4.8% |
| 16 | 29.548° | 3.02073 Å | 19.7% |

.

The characterization data of the crystal form E of the compound of formula II are as follows:

| Parameters | Method | Result |
|---|---|---|
| X-ray diffraction | 3 to 40° (2θ) | Low crystallinity, see FIG. 15; |
| Thermal analysis | DSC, 10°C/min | endothermic peaks at 192.09±3°C (onset temperature: 186.78°C, enthalpy (normalized): 80.596±0.2 J/g), 168.42±3°C (onset temperature: 158.30°C, enthalpy (normalized): 8.9656±0.2 J/g), see FIG. 16; exothermic peaks shown in the upward direction; |
| Thermal weight loss | TGA, 10°C/min | no weight loss at 30 to 160°C., see FIG. 17. |

**Example 9:** Crystal form A of the monohydrate of the 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (compound of formula II)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (0.20 g) prepared in Example 5 or 6 was added to n-heptane (50 mL), stirred, heated to 50°C, and refluxed with stirring for 96 hours. The mixture was allowed to cool naturally to room temperature, stirred for an additional 2 hours, and filtered to obtain a solid. The solid was dried

under reduced pressure to constant weight to obtain 0.18 g of the product, which was confirmed to be the crystal form A of the monohydrate of the compound of formula II, with an HPLC purity of 99.8%.

**Example 10:** 2-Amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[d]imidazole-6-carboxylate (crystal form A of the monohydrate of the compound of formula II)

The 2-amino-2-(hydroxymethyl)-1,3-propanediol salt of (S)-2-((4-(2-((4-chloro-2-fluorobenzyl)oxy)phenyl)piperidin-1-yl)methyl)-1-(oxetan-2-aminomethyl)-1H-benzo[d]imidazole-6-carboxylate (0.20 g) prepared in Example 8 was placed at 25°C/90% RH for 4 to 5 days to obtain 0.21 g of the product, which was confirmed to be the crystal form A of the monohydrate of the compound of formula II, with an HPLC purity of 99.9%.

**Example 11:** (*S*)-2-((4-(2-((4-Chloro-2-fluorobenzyl)oxy)phenyl)piperidin-1-yl)methyl)-1-(oxetan-2-aminomethyl)-1*H*-benzo[*d*]imidazole-6-carboxylic acid (crystal form I of the compound of formula I)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[d]imidazole-6-carboxylate (200 mg) prepared in Example 3 was added to acetone (1.2 mL), heated to 50°C, dissolved, cooled to approximately 25°C, slowly added dropwise with water (0.5 mL), stirred at room temperature for 24 hours, filtered to obtain a solid, and dried under reduced pressure to constant weight to obtain 150 mg of the crystal form I of the compound of formula I. XRPD: see FIG. 18. DSC: 144.32 to 154.74°C, endothermic.

The XRPD diffraction pattern analysis data for the crystal form I of the compound of formula I are shown in Table 6.

Table 6

| Number | 2θ angle | d | Relative peak intensity |
|---|---|---|---|
| 1 | 5.642° | 15.65277 Å | 15.8% |
| 2 | 8.172° | 10.81009 Å | 1.1% |
| 3 | 9.359° | 9.44155 Å | 3.7% |
| 4 | 10.898° | 8.11189 Å | 100.0% |
| 5 | 11.850° | 7.46235 Å | 3.3% |
| 6 | 13.330° | 6.63690 Å | 27.8% |
| 7 | 14.189° | 6.23705 Å | 2.2% |
| 8 | 15.212° | 5.81966 Å | 1.8% |
| 9 | 16.130° | 5.49057 Å | 3.3% |
| 10 | 16.914° | 5.23780 Å | 1.7% |
| 11 | 17.454° | 5.07679 Å | 14.0% |
| 12 | 17.995° | 4.92552 Å | 5.4% |
| 13 | 18.734° | 4.73271 Å | 1.7% |
| 14 | 19.463° | 4.55716 Å | 9.6% |
| 15 | 20.356° | 4.35911 Å | 19.1% |
| 16 | 21.300° | 4.16801 Å | 9.0% |
| 17 | 22.162° | 4.00796 Å | 9.3% |
| 18 | 22.570° | 3.93636 Å | 5.4% |
| 19 | 23.450° | 3.79052 Å | 10.9% |
| 20 | 24.396° | 3.64567 Å | 7.8% |
| 21 | 25.000° | 3.55895 Å | 13.0% |
| 22 | 25.747° | 3.45731 Å | 1.6% |
| 23 | 26.448° | 3.36728 Å | 2.2% |

(continued)

| Number | 20 angle | d | Relative peak intensity |
|---|---|---|---|
| 24 | 26.675° | 3.33912 Å | 2.2% |
| 25 | 27.513° | 3.23934 Å | 2.3% |
| 26 | 28.001° | 3.18398 Å | 1.9% |
| 27 | 29.455° | 3.02998 Å | 2.8% |
| 28 . | 30.382° | 2.93961 Å | 4.4% |

**Example 12:** (*S*)-2-((4-(2-((4-Chloro-2-fluorobenzyl)oxy)phenyl)piperidin-1-yl)methyl)-1-(oxetan-2-aminomethyl)-1*H*-benzo[*d*]imidazole-6-carboxylic acid (crystal form II of the compound of formula I)

The 2-amino-2-(hydroxymethyl)-1,3-propanediol salt of (S)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (200 mg) prepared in Example 3 was added to tetrahydrofuran (1 mL) and n-heptane (2 mL), heated to 50°C, stirred for 24 hours, cooled to room temperature, filtered to obtain a solid, and dried under reduced pressure to constant weight to obtain 180 mg of the crystal form II of the compound of formula I. XRPD: see FIG. 19. DSC: 111.96 to 129.32°C, endothermic.

The XRPD diffraction pattern analysis data for the crystal form II of the compound of formula I are shown in Table 7.

Table 7

| Number | 2θ angle | d | Relative peak intensity |
|---|---|---|---|
| 1 | 5.061° | 17.44755 Å | 1.2% |
| 2 | 5.482° | 16.10720 Å | 3.9% |
| 3 | 6.334° | 13.94231 Å | 2.6% |
| 4 | 7.104° | 12.43314 Å | 0.8% |
| 5 | 9.194° | 9.61153 Å | 2.2% |
| 6 | 10.731° | 8.23753 Å | 100.0% |
| 7 | 12.310° | 7.18439 Å | 14.6% |
| 8 | 12.754° | 6.93504 Å | 78.7% |
| 9 | 13.145° | 6.72962 Å | 18.9% |
| 10 | 15.002° | 5.90076 Å | 17.0% |
| 11 | 15.569° | 5.68711 Å | 8.7% |
| 12 | 15.951° | 5.55163 Å | 4.2% |
| 13 | 17.291° | 5.12443 Å | 2.9% |
| 14 | 18.039° | 4.91342 Å | 3.6% |
| 15 | 18.730° | 4.73375 Å | 3.5% |
| 16 | 19.277° | 4.60073 Å | 11.7% |
| 17 | 19.620° | 4.52100 Å | 26.4% |
| 18 | 20.130° | 4.40772 Å | 7.1% |
| 19 | 20.611° | 4.30580 Å | 9.1% |
| 20 | 21.017° | 4.22356 Å | 9.1% |
| 21 | 21.671° | 4.09754 Å | 8.6% |
| 22 | 22.440° | 3.95889 Å | 6.8% |
| 23 | 23.253° | 3.82225 Å | 5.9% |
| 24 | 23.872° | 3.72458 Å | 6.3% |

(continued)

| Number | 2θ angle | d | Relative peak intensity |
|---|---|---|---|
| 25 | 24.260° | 3.66589 Å | 7.7% |
| 26 | 24.805° | 3.58650 Å | 3.9% |
| 27 | 26.864° | 3.31605 Å | 1.5% |
| 28 | 27.384° | 3.25434 Å | 1.5% |
| 29 | 30.189° | 2.95801 Å | 2.2% |
| 30 | 34.314° | 2.61124 Å | 0.7% |

**Example 13:** (*S*)-2-((4-(2-((4-Chloro-2-fluorobenzyl)oxy)phenyl)piperidin-1-yl)methyl)-1-(oxetan-2-aminomethyl)-1*H*-benzo[*d*]imidazole-6-carboxylic acid (crystal form III of the compound of formula I)

The 2-amino-2-(hydroxymethyl)-1,3-propanediol salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (200 mg) prepared in Example 3 was added to methanol (1 mL) and water (1 mL), stirred at 25°C for 24 hours, cooled to room temperature, filtered to obtain a solid, and dried under reduced pressure to constant weight to obtain 163 mg of the crystal form III of the compound of formula I. XRPD: see FIG. 20. DSC: 101.13 to 121.83°C, endothermic and 136.87 to 147.61°C, endothermic.

The obtained crystal form III of the compound of formula I was the anhydrous crystal form of the compound of formula I.

The XRPD diffraction pattern analysis data for the crystal form III of the compound of formula I are shown in Table 8.

Table 8

| Number | 2θ angle | d | Relative peak intensity |
|---|---|---|---|
| 1 | 5.531° | 15.96573 Å | 2.8% |
| 2 | 6.576° | 13.42973 Å | 7.0% |
| 3 | 9.960° | 8.87373 Å | 8.0% |
| 4 | 10.535° | 8.39017 Å | 13.5% |
| 5 | 12.142° | 7.28332 Å | 100.0% |
| 6 | 13.155° | 6.72461 Å | 6.5% |
| 7 | 14.532° | 6.09039 Å | 14.1% |
| 8 | 15.201° | 5.82378 Å | 7.8% |
| 9 | 15.765° | 5.61690 Å | 21.9% |
| 10 | 16.399° | 5.40097 Å | 10.5% |
| 11 | 17.448° | 5.07852 Å | 16.1% |
| 12 | 18.985° | 4.67071 Å | 8.4% |
| 13 | 19.557° | 4.53551 Å | 71.8% |
| 14 | 20.005° | 4.43477 Å | 55.1% |
| 15 | 20.451° | 4.33925 Å | 30.5% |
| 16 | 21.217° | 4.18420 Å | 49.2% |
| 17 | 21.586° | 4.11344 Å | 20.3% |
| 18 | 22.282° | 3.98653 Å | 12.5% |
| 19 | 23.087° | 3.84931 Å | 24.0% |
| 20 | 23.492° | 3.78397 Å | 41.8% |
| 21 | 24.367° | 3.64995 Å | 11.3% |

(continued)

| Number | 2θ angle | d | Relative peak intensity |
|---|---|---|---|
| 22 | 24.665° | 3.60657 Å | 40.3% |
| 23 | 25.684° | 3.46570 Å | 14.6% |
| 24 | 26.512° | 3.35926 Å | 11.5% |
| 25 | 26.876° | 3.31461 Å | 8.5% |
| 26 | 27.994° | 3.18471 Å | 6.5% |
| 27 | 28.646° | 3.11377 Å | 11.4% |
| 28 | 29.367° | 3.03893 Å | 3.5% |
| 29 | 31.007° | 2.88177 Å | 3.7% |
| 30 | 32.856° | 2.72375 Å | 9.1% |
| 31 | 33.700° | 2.65739 Å | 5.0% |
| 32 | 34.498° | 2.59779 Å | 4.5% |
| 33 | 35.401° | 2.53355 Å | 6.1% |
| 34 | 37.509° | 2.39588 Å | 5.4% |
| 35 | 38.346° | 2.34545 Å | 3.7% |

**Effect test 1:**

The purity and hygroscopicity of the hydrochloride salt of (*S*)-2-((4-(2-((4-chloro-2-fluorobenzyl)oxy)phenyl) piperidin-1-yl)methyl)-1-(oxetan-2-aminomethyl)-1*H*-benzo[*d*]imidazole-6-carboxylate (hydrochloride salt of the compound of formula I) obtained in Example 1, the sodium salt of (*S*)-2-((4-(2-((4-chloro-2-fluorobenzyl)oxy)phenyl)piperidin-1-yl)methyl)-1-(oxetan-2-aminomethyl)-1*H*-benzo[*d*]imidazole-6-carboxylate (sodium salt of the compound of formula I) obtained in Example 2, and the crystal form A of the monohydrate of the compound of formula II obtained in Example 4 were tested.

The comparison of properties among three different salts is shown in Table 9.

Table 9

| Test items | Hydrochloride salt of the compound of formula I | Sodium salt of the compound of formula I | Tromethamine salt of the compound of formula I (Crystal form A of the monohydrate of the compound of formula II) |
|---|---|---|---|
| Medicinal crystal form | Crystal form of the compound | Crystal form of the compound | Crystal form of the hydrate of the compound |
| Purity | 94.71% | 99.9% | 99.95% |
| Process scalability | The preparation process involved acidic conditions such as hydrochloric acid, which were prone to degradation and difficult to scale up | Hygroscopic, difficult to scale up | Crystallizable, easy to scale up |
| Hygroscopicity (80% RH) | - | Highly hygroscopic, deliquescent | 0.34% Slightly hygroscopic |

**Effect test 2:** Comparison of crystal forms of the 2-amino-2-(hydroxymethyl)-1,3-propanediol salt of the compound of formula I

The crystal form A of the monohydrate of the compound of formula II, the crystal form B of the 1,4-dioxane solvate of the compound of formula II, the crystal form C of the compound of formula II, the crystal form D of the compound of

formula II, and the crystal form E of the compound of formula II obtained in Examples 4 to 8 were compared.

According to the X-ray powder diffraction patterns of each crystal form, the crystal form A of the monohydrate of the compound of formula II was found to have higher crystallinity. Furthermore, during the preparation process, it was found that the crystal form B of the 1,4-dioxane solvate of the compound of formula II and the crystal form C of the compound of formula II were unstable, prone to crystal form transformation during preparation, and exhibited poor reproducibility. The preparation processes for the crystal form D of the compound of formula II and the crystal form E of the compound of formula II involved heating to obtain the corresponding crystal forms under high-temperature conditions, which was not suitable for scale-up production (see Table 10).

Table 10

| Test items | Crystal form A of the monohydrate of the compound of formula II | Crystal form B of the 1,4-dioxane solvate of the compound of formula II | Crystal form C of the compound of formula II | Crystal form D of the compound of formula II | Crystal form E of the compound of formula II |
|---|---|---|---|---|---|
| Crystallinity | High | Low | Middle | Low | Low |
| Process scalability | Crystallizable , easy to scale up | Difficult to reproduce, hard to scale up | Difficult to reproduce, hard to scale up | High-temperature operation, unable to scale up | Heating op-eration, un-able to scale up |

**Effect test 3:**

The grindability, compressibility, hygroscopicity, and solubility of the crystal form I, crystal form II, and crystal form III of the compound of formula I obtained in Examples 11 to 13, as well as the crystal form A of the monohydrate of the compound of formula II obtained in Example 4, were tested.

The DVS test data of the crystal form I of the compound of formula I obtained in Example 11 are shown in Table 11.

Table 11

| Onset temperature | 24.9°C | | |
|---|---|---|---|
| Target humidity (% $P/P_0$) | Sorption | Desorption | Hysteresis |
| 0.0 | 0.019 | 0.013 | |
| 10.0 | 0.766 | 0.807 | 0.042 |
| 20.0 | 1.271 | 1.307 | 0.035 |
| 30.0 | 1.541 | 1.588 | 0.047 |
| 40.0 | 1.754 | 1.816 | 0.062 |
| 50.0 | 1.960 | 2.072 | 0.112 |
| 60.0 | 2.161 | 2.286 | 0.125 |
| 70.0 | 2.344 | 2.441 | 0.097 |
| 80.0 | 2.511 | 2.576 | 0.065 |
| 90.0 | 2.685 | 2.685 | |

The DVS test data of the crystal form A of the monohydrate of the compound of formula II obtained in Example 4 are as shown in Table 12.

Table 12

| Onset temperature | 24.9°C | | |
|---|---|---|---|
| Target humidity (% $P/P_0$) | Sorption | Desorption | Hysteresis |
| 0.0 | | 0.0000 | |
| 10.0 | | 0.1080 | |

(continued)

| Onset temperature | 24.9°C | | |
|---|---|---|---|
| Target humidity (% $P/P_0$) | Sorption | Desorption | Hysteresis |
| 20.0 | | 0.1542 | |
| 30.0 | | 0.1894 | |
| 40.0 | | 0.2213 | |
| 50.0 | | 0.2553 | |
| 60.0 | 0.2896 | 0.2989 | 0.0002 |
| 70.0 | 0.3160 | 0.3213 | 0.0053 |
| 80.0 | 0.3426 | 0.3490 | 0.0064 |
| 90.0 | 0.3817 | 0.3817 | |
| Cycle 2 | | | |
| 0.0 | 0.0000 | 0.0112 | |
| 10.0 | 0.1025 | 0.1186 | 0.0161 |
| 20.0 | 0.1466 | 0.1641 | 0.0175 |
| 30.0 | 0.1814 | 0.1996 | 0.0182 |
| 40.0 | 0.2141 | 0.2286 | 0.0145 |
| 50.0 | 0.2443 | 0.2611 | 0.0167 |
| 60.0 | 0.2746 | 0.2923 | 0.0177 |
| 70.0 | 0.3043 | 0.3219 | 0.0176 |
| 80.0 | 0.3458 | 0.3457 | 0.0099 |
| 90.0 | 0.3759 | 0.3759 | |

Comparative data on compound stability are shown in Table 13.

Table 13

| Test items | | Compound of formula I[a] | Crystal form I of the compound of formula I | Crystal form II of the compound of formula I | Crystal form III of the compound of formula I | Crystal form A of the monohydrate of the compound of formula II |
|---|---|---|---|---|---|---|
| Medicinal crystal form | | Amorphous | Crystal form 1, anhydrous | Crystal form 2 | Crystal form 3 | Monohydrate |
| Purity[b] | Onset | 99.15% | 99.27% | 99.73% | 99.25% | 99.95% |
| | 80°C (1 day) | 70.18%[c] amorphous | 91.81% | 80.43% transformed to amorphous | 83.43% transformed to amorphous | 99.35% retained the crystal form |
| | 60°C (7 day) | 99.15% | - | - | - | 99.90% |
| | 2 to 8°C (6 months) | 82.49% | - | - | - | 99.90% |
| Compressibility (20 Mp) | | Significant reduction in crystallinity, amorphization | Crystal form I, significant reduction in crystallinity | Crystal form II, significant reduction in crystallinity | Crystal form III, no significant reduction in crystallinity | Crystal form unchanged |

(continued)

| Test items | Compound of formula I[a] | Crystal form I of the compound of formula I | Crystal form II of the compound of formula I | Crystal form III of the compound of formula I | Crystal form A of the monohydrate of the compound of formula II |
|---|---|---|---|---|---|
| Grindability (mortar 5 min) | Amorphous, purity decreased by 6.5% | 99.25% transformed to amorphous | 99.7% transformed to amorphous | 98.65% transformed to amorphous | Crystalline form unchanged, purity unchanged |
| Crystallinity | - | High | High | Middle | High |
| Process scalability | | Crystallizable, easy to scale up | Difficult to reproduce, hard to scale up | Difficult to reproduce, hard to scale up | Easy to scale up |
| Hygroscopicity (80% RH) | 0.25% (10 to 90% RH linear reversible) | 2.5% highly hygroscopic | | | 0.34% (10 to 90% RH linear reversible) |
| Solubility | Less than 0.001 | | | | 0.06 mg/mL |
| mg/mL | | | | | |

Note:
[a]The preparation method was the same as that described in Example 3 of Patent CN202110334388.
[b]In the purity test, the sample was placed in an oven at the temperature set in the table, and the purity was tested after the specified time.
[c]The result after being placed at 80 °C for 30 days.

It can be seen from the above table that the compound of formula I has poor thermal stability, will degrade under heating or room temperature, and has poor processability, and obvious degradation after grinding and tableting.

Among them, the crystal form I, the crystal form II, and the crystal form III of the compound of formula I have poor processability, and as shown in FIG. 21, FIG. 22, and FIG. 23. Compared with the crystal form I, the crystal form II, and the crystal form III of the compound of formula I, the crystal form I, the crystal form II, and the crystal form III after tableting, the crystal form I, the crystal form II, and the crystal form III after grinding for 5 minutes, and the crystal form I, the crystal form II, and the crystal form III after grinding for 2 minutes all have a phenomenon of decreasing crystallinity in different degrees. In contrast, as shown in FIG. 24 and FIG. 25, the crystallinity of the crystal form A of the monohydrate of the compound of formula II is basically stable after the first tableting at 20 MPa, the second tableting at 20 MPa, tableting at 40 MPa, and grinding for 1 minute, 3 minutes, and 5 minutes. It can be seen that the crystal form A of the monohydrate of the compound of formula II has good processability. Moreover, the crystal form A of the monohydrate of the compound of formula II is stable, has good experimental reproducibility, has good hygroscopicity and solubility, and has broad medicinal prospects.

**Effect test 4: Pharmacokinetic evaluation in rats**

Rats were used as test animals, and the plasma drug concentrations at different time points after gavage administration of the test sample were measured. The pharmacokinetic behavior of the compound of the present disclosure in rats was studied to evaluate its metabolic characteristics. For each experimental group, 3 rats with similar body weights were selected and orally administered a single dose of 10 mg/kg. Blood samples were collected at 15 min, 30 min, 1 hour, 2 hours, 4 hours, 6 hours, 8 hours, 12 hours, and 24 hours after administration of the animal. The compound content in plasma was determined by LC-MS/MS analysis method with a lower limit of quantification of 20 ng/mL. Pharmacokinetic parameter calculations were performed using metabolic kinetic data analysis software WinNonlin 7.0 by non-compartmental analysis (NCA) based on plasma concentration data.

Experimental drugs: Crystal form A of the monohydrate of the compound of formula II obtained in Example 4 and the compound of formula I (free acid).

Drug preparation:

A test sample with a final preparation concentration (based on the content of the compound of formula I) of 1.0

mg/mL was prepared for oral administration, using a 0.5% CMC Na aqueous solution as the preparation solvent. After preparation, a milky white homogeneous suspension was obtained.

Administration: After overnight fasting (12 hours), rats were administered by gavage at a dose of 10 mg/kg.

Procedure: Rats were administered by gavage, and blood samples were collected from the tail vein before administration and at 15 min, 30 min, 1 hour, 2 hours, 4 hours, 6 hours, 8 hours, 12 hours, and 24 hours after administration. The blood samples were placed in heparinized sample tubes, and centrifuged at 4°C and 3500 rpm for 10 minutes to separate plasma, which was then stored at -20°C. Food was provided 2 hours after administration.

Determination of the content of the compound to be tested in rat plasma after gavage administration: After thawing plasma samples at room temperature, 300 μL of internal standard (terfenadine 200 ng/mL, acetonitrile) was added to each sample. The mixtures were vortexed for 1 min and then centrifuged at 4°C and 15400 rpm for 10 min. The supernatant was diluted 20-fold with 80% acetonitrile-water and then injected for LC/MS/MS analysis.

| **Compound** | **Rat test (Rat PK), oral (PO), 10 mg/kg** | | | | |
|---|---|---|---|---|---|
| | $T_{max}$ | $C_{max}$ | $AUC_{0-t}$ | $\mathbf{AUC_{0-\infty}}$ | $t_{1/2}$ |
| | (h) | (ng/mL) | (ng·h/mL) | (ng·h/mL) | (h) |
| Crystal form A of the monohydrate of the compound of formula II | 0.410 | 410 | 765 | 772 | 1.59 |
| Compound of formula I (free acid) | 0.667 | 154 | 396 | 401 | 1.09 |

As shown in the above table, the crystal form A of the monohydrate of the compound of formula II in the present disclosure has significantly better oral absorption than the compound of formula I (free acid).

## Claims

1. A 2-amino-2-(hydroxymethyl)-1,3-propanediol salt of a compound of formula I or a solvate thereof;

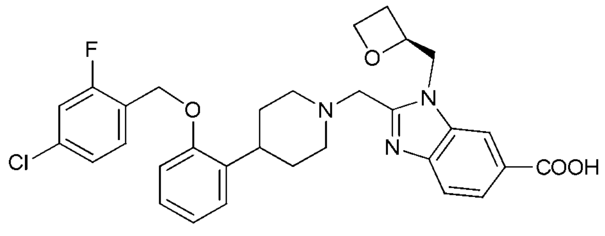

I

.

2. The 2-amino-2-(hydroxymethyl)-1,3-propanediol salt of the compound of formula I or the solvate thereof according to claim 1, wherein the 2-amino-2-(hydroxymethyl)-1,3-propanediol salt of the compound of formula I or the solvate thereof satisfies one or more of the following conditions:

(1) the 2-amino-2-(hydroxymethyl)-1,3-propanediol salt of the compound of formula I is a compound of formula II,

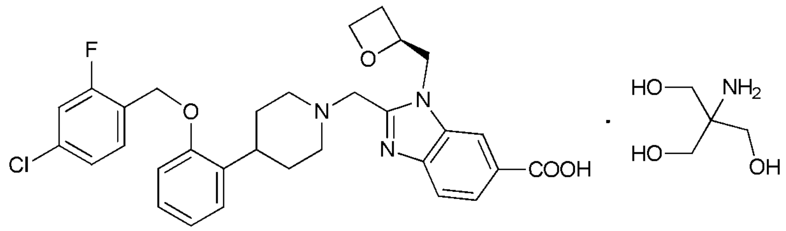

II

;

and

(2) in the solvate of the 2-amino-2-(hydroxymethyl)-1,3-propanediol salt of the compound of formula I, the solvent is water or 1,4-dioxane; in the solvate of the 2-amino-2-(hydroxymethyl)-1,3-propanediol salt of the compound of formula I, the molar ratio of the 2-amino-2-(hydroxymethyl)-1,3-propanediol salt of the compound of formula I to the solvent is preferably 1:(1-0.5), for example, the molar ratio of the 2-amino-2-(hydroxymethyl)-1,3-propanediol salt of the compound of formula I to water is 1:1, and for another example, the molar ratio of the 2-amino-2-(hydroxymethyl)-1,3-propanediol salt of the compound of formula I to 1,4-dioxane is 1:0.5.

**3.** A crystal form of a compound of formula II or a solvate thereof, wherein the crystal form of the compound of formula II or the solvate thereof is a crystal form A of a monohydrate of a compound of formula II, a crystal form B of a 1,4-dioxane solvate of a compound of formula II, a crystal form C of a compound of formula II, a crystal form D of a compound of formula II, or a crystal form E of a compound of formula II;

wherein the monohydrate of the compound of formula II has a structure of:

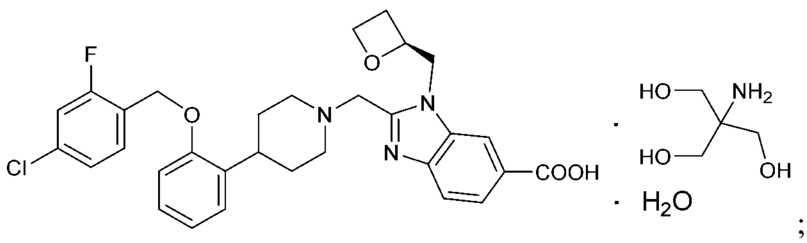

;

the 1,4-dioxane solvate of the compound of formula II has a structure of:

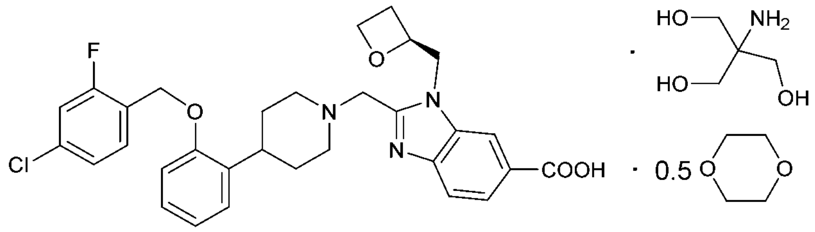

;

the compound of formula II has a structure of:

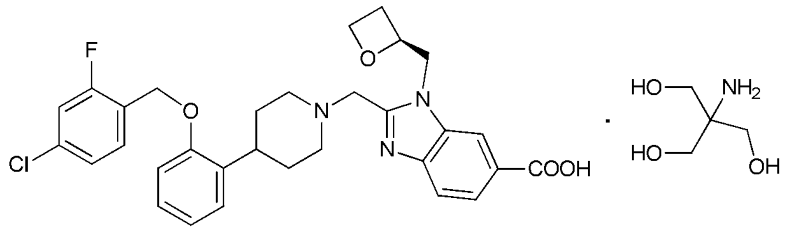

II

;

the crystal form A of the monohydrate of the compound of formula II has an X-ray powder diffraction pattern obtained using Cu-Kα radiation and expressed in terms of 2θ angles, comprising diffraction peaks at 4.1±0.2°, 8.2±0.2°, 10.7±0.2°, 12.3±0.2°, and 20.7±0.2°;
the crystal form B of the 1,4-dioxane solvate of the compound of formula II has an X-ray powder diffraction pattern obtained using Cu-Kα radiation and expressed in terms of 2θ angles, comprising diffraction peaks at 3.4±0.2°, 7.4±0.2°, 11.2±0.2°, and 14.6±0.2°;
the crystal form C of the compound of formula II has an X-ray powder diffraction pattern obtained using Cu-Kα radiation and expressed in terms of 2θ angles, comprising diffraction peaks at 3.7±0.2°, 7.5±0.2°, 9.7±0.2°, and 15.5±0.2°;
the crystal form D of the compound of formula II has an X-ray powder diffraction pattern obtained using Cu-Kα radiation and expressed in terms of 2θ angles, comprising diffraction peaks at 3.9±0.2°, 8.1±0.2°, 10.6±0.2°,

and 14.9±0.2°;
the crystal form E of the compound of formula II has an X-ray powder diffraction pattern obtained using Cu-Kα radiation and expressed in terms of 2θ angles, comprising diffraction peaks at 3.7±0.2°, 7.6±0.2°, 9.8±0.2°, and 14.7±0.2°.

**4.** The crystal form of the compound of formula II or the solvate thereof according to claim 3, wherein the crystal form of the compound of formula II satisfies one or more of the following conditions:

(1) the X-ray powder diffraction pattern of the crystal form A of the monohydrate of the compound of formula II, obtained using Cu-Kα radiation and expressed in terms of 2θ angles, further comprises diffraction peaks at one or more of the following positions: 14.1±0.2°, 14.9±0.2°, 20.5±0.2°, and 21.8±0.2°;
preferably, the X-ray powder diffraction pattern of the crystal form A of the monohydrate of the compound of formula II, obtained using Cu-Kα radiation and expressed in terms of 2θ angles, comprises diffraction peaks at 4.1 ±0.2°, 8.2±0.2°, 10.7±0.2°, 12.3±0.2°, 14.1±0.2°, 14.9±0.2°, 20.5±0.2°, and 21.8±0.2°;
(2) the X-ray powder diffraction pattern of the crystal form B of the 1,4-dioxane solvate of the compound of formula II, obtained using Cu-Kα radiation and expressed in terms of 2θ angles, further comprises diffraction peaks at one or more of the following positions: 8.2±0.2°, 12.3±0.2°, 13.7±0.2°, 16.1±0.2°, 20.0±0.2°, 22.0±0.2°, 22.5 ±0.2°, and 29.3±0.2°;
preferably, the X-ray powder diffraction pattern of the crystal form B of the 1,4-dioxane solvate of the compound of formula II, obtained using Cu-Kα radiation and expressed in terms of 2θ angles, comprises diffraction peaks at 3.4 ±0.2°, 7.4±0.2°, 8.2±0.2°, 11.2±0.2°, 12.3±0.2°, 13.7±0.2°, 14.6±0.2°, 16.1±0.2°, 20.0±0.2°, 22.0±0.2°, 22.5±0.2°, and 29.3±0.2°;
(3) the X-ray powder diffraction pattern of the crystal form C of the compound of formula II, obtained using Cu-Kα radiation and expressed in terms of 20 angles, further comprises diffraction peaks at one or more of the following positions: 11.3±0.2°, 12.3±0.2°, 14.4±0.2°, 14.7±0.2°, 17.2±0.2°, 20.5±0.2°, 21.8±0.2°, and 29.5±0.2°;
preferably, the X-ray powder diffraction pattern of the crystal form C of the compound of formula II, obtained using Cu-Kα radiation and expressed in terms of 20 angles, comprises diffraction peaks at 3.7±0.2°, 7.5±0.2°, 9.7 ±0.2°, 11.3±0.2°, 12.3±0.2°, 14.4±0.2°, 14.7±0.2°, 15.5±0.2°, 17.2±0.2°, 20.5±0.2°, 21.8±0.2, and 29.5 ±0.2°;
(4) the X-ray powder diffraction pattern of the crystal form D of the compound of formula II, obtained using Cu-Kα radiation and expressed in terms of 20 angles, further comprises diffraction peaks at one or more of the following positions: 10.2±0.2°, 10.6±0.2°, 14.3±0.2°, 17.8±0.2°, 20.0±0.2°, and 20.2±0.2°;
preferably, the X-ray powder diffraction pattern of the crystal form D of the compound of formula II, obtained using Cu-Kα radiation and expressed in terms of 20 angles, comprises diffraction peaks at 3.9±0.2°, 8.1±0.2°, 10.2 ±0.2°, 10.6±0.2°, 14.3±0.2°, 14.9±0.2°, 17.8±0.2°, 20.0±0.2°, and 20.2±0.2°; and
(5) the X-ray powder diffraction pattern of the crystal form E of the compound of formula II, obtained using Cu-Kα radiation and expressed in terms of 20 angles, further comprises diffraction peaks at one or more of the following positions: 11.2±0.2°, 15.6±0.2°, 17.4±0.2°, 19.8±0.2°, 20.3±0.2°, 21.9±0.2°, 29.5±0.2°, and 19.0±0.2°;
preferably, the X-ray powder diffraction pattern of the crystal form E of the compound of formula II, obtained using Cu-Kα radiation and expressed in terms of 20 angles, comprises diffraction peaks at 3.7±0.2°, 7.6±0.2°, 9.8 ±0.2°, 11.2±0.2°, 14.7±0.2°, 15.6±0.2°, 17.4±0.2°, 19.8±0.2°, 20.3±0.2°, 21.9±0.2°, and 29.5±0.2°.

**5.** The crystal form of the compound of formula II or the solvate thereof according to claim 4, wherein the crystal form of the compound of formula II satisfies one or more of the following conditions:

(1) the X-ray powder diffraction pattern of the crystal form A of the monohydrate of the compound of formula II, obtained using Cu-Kα radiation and expressed in terms of 2θ angles, comprises diffraction peaks as shown in Table 1:

Table 1

| Number | 2θ angle | d | Relative peak intensity |
|---|---|---|---|
| 1 | 4.114° | 21.46041 Å | 19.8% |
| 2 | 8.295° | 10.65090 Å | 91.1% |
| 3 | 10.703° | 8.25895 Å | 80.6% |
| 4 | 12.388° | 7.13911 Å | 100.0% |

(continued)

| Number | 2θ angle | d | Relative peak intensity |
|---|---|---|---|
| 5 | 14.151° | 6.25379 Å | 64.9% |
| 6 | 14.941° | 5.92472 Å | 65.2% |
| 7 | 15.954° | 5.55077 Å | 31.3% |
| 8 | 16.566° | 5.34683 Å | 13.9% |
| 9 | 17.086° | 5.18547 Å | 16.0% |
| 10 | 17.948° | 4.93836 Å | 18.9% |
| 11 | 18.429° | 4.81041 Å | 17.4% |
| 12 | 19.019° | 4.66256 Å | 36.0% |
| 13 | 19.916° | 4.45440 Å | 19.7% |
| 14 | 20.248° | 4.38230 Å | 21.0% |
| 15 | 20.530° | 4.32259 Å | 48.4% |
| 16 | 20.768° | 4.27354 Å | 94.2% |
| 17 | 21.362° | 4.15615 Å | 40.2% |
| 18 | 21.868° | 4.06113 Å | 70.7% |
| 19 | 22.405° | 3.96502 Å | 28.4% |
| 20 | 23.059° | 3.85389 Å | 18.2% |
| 21 | 23.528° | 3.77811 Å | 12.6% |
| 22 | 23.874° | 3.72426 Å | 22.6% |
| 23 | 24.612° | 3.61423 Å | 5.5% |
| 24 | 25.081° | 3.54767 Å | 23.8% |
| 25 | 25.383° | 3.50611 Å | 14.5% |
| 26 | 25.899° | 3.43736 Å | 42.5% |
| 27 | 26.264° | 3.39049 Å | 30.7% |
| 28 | 26.667° | 3.34010 Å | 18.2% |
| 29 | 27.176° | 3.27878 Å | 4.8% |
| 30 | 27.646° | 3.22405 Å | 11.1% |
| 31 | 27.968° | 3.18764 Å | 4.4% |
| 32 | 28.847° | 3.09251 Å | 7.2% |
| 33 | 29.734° | 3.00219 Å | 20.1% |
| 34 | 30.268° | 2.95045 Å | 2.4% |
| 35 | 30.988° | 2.88351 Å | 8.4% |
| 36 | 31.535° | 2.83476 Å | 8.1% |
| 37 | 32.098° | 2.78633 Å | 11.5% |
| 38 | 32.770° | 2.73066 Å | 2.7% |
| 39 | 33.246° | 2.69269 Å | 1.9% |
| 40 | 34.317° | 2.61104 Å | 9.7% |
| 41 | 34.659° | 2.58607 Å | 4.3% |
| 42 | 35.356° | 2.53667 Å | 9.7% |
| 43 | 36.387° | 2.46713 Å | 2.7% |

(continued)

| Number | 2θ angle | d | Relative peak intensity |
|---|---|---|---|
| 44 | 37.305° | 2.40846 Å | 4.9% |
| 45 | 37.817° | 2.37704 Å | 9.3% |
| 46 | 38.537° | 2.33425 Å | 2.8% |
| 47 | 39.257° | 2.29310 Å | 2.1% |

preferably, the X-ray powder diffraction pattern of the crystal form A of the monohydrate of the compound of formula II obtained using Cu-Kα radiation is substantially as shown in FIG. 1;
(2) the crystal form A of the monohydrate of the compound of formula II has a differential scanning calorimetry curve showing endothermic peaks at 129.98±3°C and 196.25±3°C;
preferably, the differential scanning calorimetry curve of the crystal form A of the monohydrate of the compound of formula II is as shown in FIG. 2;
(3) the crystal form A of the monohydrate of the compound of formula II has a thermogravimetric analysis curve showing a weight loss of 2.468% within the temperature range of 30±3°C to 135±3°C;
preferably, the thermogravimetric analysis curve of the crystal form A of the monohydrate of the compound of formula II is as shown in FIG. 3;
(4) the X-ray powder diffraction pattern of the crystal form B of the 1,4-dioxane solvate of the compound of formula II, obtained using Cu-Kα radiation and expressed in terms of 2θ angles, comprises diffraction peaks as shown in Table 2:

Table 2

| Number | 2θ angle | d | Relative peak intensity |
|---|---|---|---|
| 1 | 3.435° | 25.70386 Å | 5.8% |
| 2 | 7.436° | 11.87902 Å | 35.0% |
| 3 | 8.210° | 10.76082 Å | 25.6% |
| 4 | 8.923° | 9.90266 Å | 10.4% |
| 5 | 9.734° | 9.07927 Å | 6.8% |
| 6 | 11.232° | 7.87142 Å | 8.5% |
| 7 | 12.353° | 7.15933 Å | 31.7% |
| 8 | 13.769° | 6.42629 Å | 6.7% |
| 9 | 14.109° | 6.27207 Å | 11.7% |
| 10 | 14.602° | 6.06125 Å | 13.4% |
| 11 | 14.894° | 5.94310 Å | 20.1% |
| 12 | 16.133° | 5.48967 Å | 42.0% |
| 13 | 17.912° | 4.94809 Å | 11.5% |
| 14 | 20.063° | 4.42215 Å | 31.5% |
| 15 | 20.685° | 4.29067 Å | 50.3% |
| 16 | 22.002° | 4.03663 Å | 100.0% |
| 17 | 22.533° | 3.94269 Å | 50.5% |
| 18 | 23.628° | 3.76247 Å | 9.8% |
| 19 | 25.802° | 3.45007 Å | 8.8% |
| 20 | 29.387° | 3.03690 Å | 15.7% |
| 21 | 31.178° | 2.86638 Å | 9.9% |

; preferably, the X-ray powder diffraction pattern of the crystal form B of the 1,4-dioxane solvate of the compound of formula II obtained using Cu-Kα radiation is substantially as shown in FIG. 6;
(5) the crystal form B of the 1,4-dioxane solvate of the compound of formula II has a differential scanning calorimetry curve showing endothermic peaks at 107.2±3°C and 186.6±3°C;
preferably, the differential scanning calorimetry curve of the crystal form B of the 1,4-dioxane solvate of the compound of formula II is as shown in FIG. 7;
(6) the crystal form B of the 1,4-dioxane solvate of the compound of formula II has a thermogravimetric analysis curve showing a weight loss of 2.029% within the temperature range of 33±3°C to 150±3°C;
preferably, the thermogravimetric analysis curve of the crystal form B of the 1,4-dioxane solvate of the compound of formula II is as shown in FIG. 8;
(7) the X-ray powder diffraction pattern of the crystal form C of the compound of formula II, obtained using Cu-Kα radiation and expressed in terms of 2θ angles, comprises diffraction peaks as shown in Table 3:

Table 3

| Number | 2θ angle | d | Relative peak intensity |
|---|---|---|---|
| 1 | 3.722° | 23.71970 Å | 63.7% |
| 2 | 7.544° | 11.70969 Å | 53.0% |
| 3 | 8.211° | 10.75927 Å | 19.3% |
| 4 | 9.742° | 9.07174 Å | 100.0% |
| 5 | 11.335° | 7.80031 Å | 21.0% |
| 6 | 12.330° | 7.17280 Å | 26.5% |
| 7 | 14.468° | 6.11713 Å | 32.1% |
| 8 | 14.768° | 5.99349 Å | 55.2% |
| 9 | 15.599° | 5.67635 Å | 64.4% |
| 10 | 17.280° | 5.12770 Å | 41.6% |
| 11 | 17.708° | 5.00461 Å | 11.8% |
| 12 | 19.738° | 4.49433 Å | 42.6% |
| 13 | 20.374° | 4.35536 Å | 57.2% |
| 14 | 20.556° | 4.31726 Å | 62.7% |
| 15 | 21.880° | 4.05882 Å | 65.0% |
| 16 | 29.519° | 3.02364 Å | 23.8% |

; preferably, the X-ray powder diffraction pattern of the crystal form C of the compound of formula II obtained using Cu-Kα radiation is substantially as shown in FIG. 9;
(8) the crystal form C of the compound of formula II has a differential scanning calorimetry curve showing an endothermic peak at 196.9±3°C;
preferably, the differential scanning calorimetry curve of the crystal form C of the compound of formula II is as shown in FIG. 10;
(9) the crystal form C of the compound of formula II has a thermogravimetric analysis curve showing no weight loss within the temperature range of 30±3°C to 180±3°C;
preferably, the thermogravimetric analysis curve of the crystal form C of the compound of formula II is as shown in FIG. 11;
(10) the crystal form C of the compound of formula II is a non-solvated crystal form;
(11) the X-ray powder diffraction pattern of the crystal form D of the compound of formula II, obtained using Cu-Kα radiation and expressed in terms of 2θ angles, comprises diffraction peaks as shown in Table 4:

Table 4

| Number | 2θ angle | d | Relative peak intensity |
|---|---|---|---|
| 1 | 3.959° | 22.29885 Å | 94.9% |
| 2 | 8.136° | 10.85780 Å | 86.6% |

(continued)

| Number | 2θ angle | d | Relative peak intensity |
|---|---|---|---|
| 3 | 10.245° | 8.62776 Å | 64.7% |
| 4 | 10.697° | 8.26398 Å | 78.4% |
| 5 | 14.334° | 6.17399 Å | 47.7% |
| 6 | 14.954° | 5.91937 Å | 81.6% |
| 7 | 15.904° | 5.56799 Å | 35.8% |
| 8 | 17.164° | 5.16195 Å | 21.4% |
| 9 | 17.898° | 4.95198 Å | 40.1% |
| 10 | 18.073° | 4.90432 Å | 35.0% |
| 11 | 20.091° | 4.41609 Å | 61.8% |
| 12 | 20.264° | 4.37868 Å | 100.0% |
| 13 | 21.600° | 4.11090 Å | 68.3% |
| 14 | 23.076° | 3.85110 Å | 15.4% |

; preferably, the X-ray powder diffraction pattern of the crystal form D of the compound of formula II obtained using Cu-Kα radiation is substantially as shown in FIG. 12;

(12) the crystal form D of the compound of formula II has a differential scanning calorimetry curve showing endothermic peaks at 178.3±3°C and 194.0±3°C;

preferably, the differential scanning calorimetry curve of the crystal form D of the compound of formula II is as shown in FIG. 13;

(13) the crystal form D of the compound of formula II has a thermogravimetric analysis curve showing a weight loss of 0.59% within the temperature range of 30±3°C to 101±3°C, and a weight loss of 0.81% within the temperature range of 140±3°C to 180±3°C;

preferably, the thermogravimetric analysis curve of the crystal form D of the compound of formula II is as shown in FIG. 14;

(14) the crystal form D of the compound of formula II is a non-solvated crystal form;

(15) the X-ray powder diffraction pattern of the crystal form E of the compound of formula II, obtained using Cu-Kα radiation and expressed in terms of 20 angles, comprises diffraction peaks as shown in Table 5:

Table 5

| Number | 2θ angle | d | Relative peak intensity |
|---|---|---|---|
| 1 | 3.725° | 23.70211 Å | 48.7% |
| 2 | 7.606° | 11.61356 Å | 44.4% |
| 3 | 9.834° | 8.98712 Å | 100.0% |
| 4 | 11.297° | 7.82653 Å | 27.8% |
| 5 | 13.430° | 6.58762 Å | 5.9% |
| 6 | 14.443° | 6.12784 Å | 32.8% |
| 7 | 14.722° | 6.01220 Å | 68.7% |
| 8 | 15.651° | 5.65749 Å | 75.3% |
| 9 | 17.416° | 5.08775 Å | 43.8% |
| 10 | 19.037° | 4.65818 Å | 9.2% |
| 11 | 19.892° | 4.45989 Å | 58.3% |
| 12 | 20.382° | 4.35373 Å | 55.8% |
| 13 | 21.980° | 4.04064 Å | 46.1% |
| 14 | 23.806° | 3.73464 Å | 7.1% |

(continued)

| Number | 2θ angle | d | Relative peak intensity |
|---|---|---|---|
| 15 | 26.722° | 3.33344 Å | 4.8% |
| 16 | 29.548° | 3.02073 Å | 19.7% |

; preferably, the X-ray powder diffraction pattern of the crystal form E of the compound of formula II obtained using Cu-Kα radiation is substantially as shown in FIG. 15;

(16) the crystal form E of the compound of formula II has a differential scanning calorimetry curve showing an endothermic peak at 192.0±3°C;

preferably, the differential scanning calorimetry curve of the crystal form E of the compound of formula II is as shown in FIG. 16;

(17) the crystal form E of the compound of formula II has a thermogravimetric analysis curve showing no weight loss within the range of 30±3°C to 160±3°C;

preferably, the thermogravimetric analysis curve of the crystal form E of the compound of formula II is as shown in FIG. 17; and

(18) the crystal form E of the compound of formula II is a non-solvated crystal form.

**6.** A single crystal of a monohydrate of a compound of formula II, wherein the single crystal has the following unit cell parameters: space group $P2_12_12_1$; a=6.13580(4)Å, α=90°, b=12.99957(8)Å, β=90°, c=42.4637(3)Å, γ=90°, unit cell volume=3387.02(4)$Å^3$, number of asymmetric units in the unit cell Z=4, crystal density=1.379 mg/$m^3$;
wherein the monohydrate of the compound of formula II has a structure of:

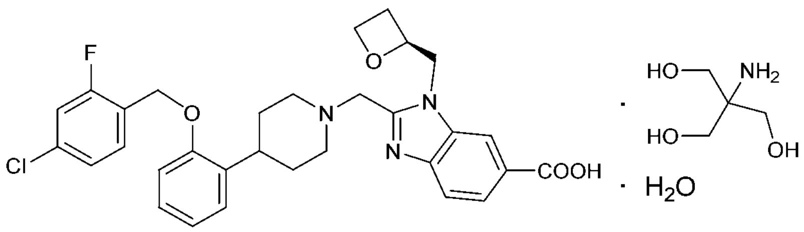

;

preferably, the single crystal of the monohydrate of the compound of formula II is orthorhombic.

**7.** A preparation method for the crystal form of the compound of formula II or the solvate thereof according to any one of claims 3 to 5, comprising the following steps:

(1) mixing the 2-amino-2-(hydroxymethyl)-1,3-propanediol salt of the compound of formula I according to claim 1 or 2 with a solvent to obtain a mixture;

(2) filtering the mixture and drying to obtain the crystal form of the compound of formula II or the solvate thereof;

wherein

when the solvent is an alcohol solvent, the crystal form A of the monohydrate of the compound of formula II is obtained;

when the solvent is 1,4-dioxane, the crystal form B of the 1,4-dioxane solvate of the compound of formula II is obtained;

when the solvent is tetrahydrofuran, the crystal form C of the compound of formula II is obtained;

preferably, the preparation method satisfies one or more of the following conditions:

(1) in the preparation method, the alcohol solvent is methanol;

(2) in the preparation method, the mixing is performed with stirring to form a suspension;

(3) in the preparation method, the mixture obtained in step (1) is a suspension;

(4) in the preparation method, the drying is performed under reduced pressure or under normal pressure;

(5) in the preparation method, when the crystal form A of the monohydrate of the compound of formula II is obtained, the mass-to-volume ratio of the compound of formula II to the solvent is 1:(1 to 100) g/mL, for example, 1:30 g/mL;

(6) in the preparation method, when the crystal form A of the monohydrate of the compound of formula II is obtained, the temperature for mixing is 0 to 150°C, for example, the temperature for mixing is 20 to 80°C, preferably being heated to reflux;
(7) in the preparation method, when the crystal form A of the monohydrate of the compound of formula II is obtained, the filtering is performed at room temperature, for example, filtering after cooling the mixture to room temperature;
(8) in the preparation method, when the crystal form B of the 1,4-dioxane solvate of the compound of formula II is obtained, the mass-to-volume ratio of the compound of formula II to the solvent is 1:(10 to 50) g/mL, for example, 1:20 g/mL;
(9) in the preparation method, when the crystal form B of the 1,4-dioxane solvate of the compound of formula II is obtained, the temperature for mixing is room temperature, wherein the room temperature is 20 to 30°C, for example, 25°C;
(10) in the preparation method, when the crystal form B of the 1,4-dioxane solvate of the compound of formula II is obtained, the mixing is performed with stirring;
(11) in the preparation method, when the crystal form B of the 1,4-dioxane solvate of the compound of formula II is obtained, the filtering is performed at room temperature;
(12) in the preparation method, when the crystal form C of the compound of formula II is obtained, the mass-to-volume ratio of the compound of formula II to the solvent is 1:(5 to 20) g/mL, for example, 1:10 g/mL;
(13) in the preparation method, when the crystal form C of the compound of formula II is obtained, the temperature for mixing is 45 to 55°C, for example, 25 to 50°C, preferably 50°C; and
(14) in the preparation method, when the crystal form C of the compound of formula II is obtained, the mixing is performed with stirring.

**8.** The preparation method according to claim 7, wherein the preparation method further comprises the following steps:

step (1a): mixing the compound of formula I with solvent A to obtain mixture A;
step (1b): mixing 2-amino-2-(hydroxymethyl)-1,3-propanediol with solvent B to obtain mixture B;
steps (1a) and (1b) are in no particular order;
step (2): mixing the mixture A and the mixture B, crystallizing, filtering, and drying to obtain the compound of formula II;
preferably, the preparation method satisfies one or more of the following conditions:

(1) in step (1a), the solvent A is one or more of alcohol solvents, ester solvents, ether solvents, ketone solvents, chlorinated hydrocarbon solvents, aromatic hydrocarbon solvents, nitrile solvents, alkane solvents, and nitrogen-containing compound solvents; the alcohol solvent is, for example, methanol, ethanol, or isopropanol; the ether solvent is, for example, methyl tert-butyl ether, isopropyl ether, tetrahydrofuran, or 2-methyltetrahydrofuran; the nitrogen-containing compound solvent is, for example, N-methylpyrrolidone; the ester solvent is, for example, methyl formate, ethyl formate, ethyl acetate, or isopropyl acetate; further preferably, the solvent A is methanol, ethanol, acetone, ethyl acetate, a mixed solution of *n*-heptane and ethyl acetate, a mixed solution of n-heptane and tetrahydrofuran, a mixed solution of methanol and water, a mixed solution of acetone and water, a mixed solution of ethanol and water, a mixed solution of tetrahydrofuran and water, "a mixed solution of methanol, water, and dimethyl sulfoxide", "a mixed solution of 2-methyltetrahydrofuran, ethyl acetate, and methanol", "a mixed solution of ethyl acetate, tetrahydrofuran, and water", or "a mixed solution of ethyl acetate, 2-methyltetrahydrofuran, and water";
furthermore, in step (1a), the solvent A is an alcohol solvent, for example, the solvent A is methanol;
(2) in step (1a), the mass-to-volume ratio of the compound of formula I to the solution A is 10:(10 to 500) g/mL, for example, 10:200 g/mL;
(3) in step (1a), the temperature for mixing is 10 to 160°C, preferably 20 to 30°C;
(4) in step (1a), the mixture A is further filtered after the mixing;
(5) in step (1b), the solvent B is one or more of water, alcohol solvents, and sulfur-containing compound solvents, wherein the alcohol solvent is, for example, ethanol or methanol, and the sulfur-containing compound solvent may be dimethyl sulfoxide;
preferably, in step (1b), the solvent B is water;
(6) in step (1b), the mass ratio of 2-amino-2-(hydroxymethyl)-1,3-propanediol to the solvent B is 1:(1 to 260), preferably 3.2:800;
(7) in step (2), the mixing is performed by adding the mixture B dropwise to the mixture A;
(8) in step (2), the temperature for crystallizing is 20 to 30°C;
(9) in step (2), the filtering comprises the steps of filtering, washing, and pH adjustment;

(10) in step (2), the drying is performed at 45°C under vacuum; and

(11) in step (1a), when the solvent A is "a mixed solution of 2-methyltetrahydrofuran, ethyl acetate, and methanol", the volume ratio of 2-methyltetrahydrofuran, ethyl acetate, and methanol is 5:5:2.

**9.** A preparation method for the crystal form D of the compound of formula II according to any one of claims 3 to 5, wherein the method comprises the following steps: heating the crystal form C of the compound of formula II according to any one of claims 3 to 5 or the crystal form B of the 1,4-dioxane solvate of the compound of formula II according to any one of claims 3 to 5 to 160°C for 5 to 10 minutes to obtain the crystal form D of the compound of formula II.

**10.** A preparation method for the crystal form E of the compound of formula II according to any one of claims 3 to 5, wherein the method comprises the following steps: heating the crystal form B of the 1,4-dioxane solvate of the compound of formula II according to any one of claims 3 to 5 or the crystal form C of the compound of formula II according to any one of claims 3 to 5 to 80 to 100°C for 24 to 96 hours to obtain the crystal form E of the compound of formula II.

**11.** A pharmaceutical composition, comprising the 2-amino-2-(hydroxymethyl)-1,3-propanediol salt of the compound of formula I or the solvate thereof according to any one of claims 1 to 2, and a pharmaceutically acceptable carrier; preferably, the 2-amino-2-(hydroxymethyl)-1,3-propanediol salt of the compound of formula I or the solvate thereof is the single crystal of the monohydrate of the compound of formula II according to claim 6 or the crystal form of the compound of formula II or the solvate thereof according to any one of claims 3 to 5.

**12.** Use of the pharmaceutical composition according to claim 11 or the 2-amino-2-(hydroxymethyl)-1,3-propanediol salt of the compound of formula I or the solvate thereof according to any one of claims 1 to 2 in the manufacture of a medicament for preventing and/or treating metabolism-related diseases;

preferably, the 2-amino-2-(hydroxymethyl)-1,3-propanediol salt of the compound of formula I or the solvate thereof is the single crystal of the monohydrate of the compound of formula II according to claim 6 or the crystal form of the compound of formula II or the solvate thereof according to any one of claims 3 to 5;

in the use, the metabolism-related diseases may be selected from any one of glucose intolerance, hyperglycemia, dyslipidemia, type 1 diabetes, type 2 diabetes, hypertriglyceridemia, insulin resistance, impaired glucose tolerance, diabetic dyslipidemia, hyperlipidemia, arteriosclerosis, atherosclerosis, hypertension, obesity, non-alcoholic fatty liver disease, non-alcoholic steatohepatitis, liver fibrosis, cirrhosis, and lethargy.

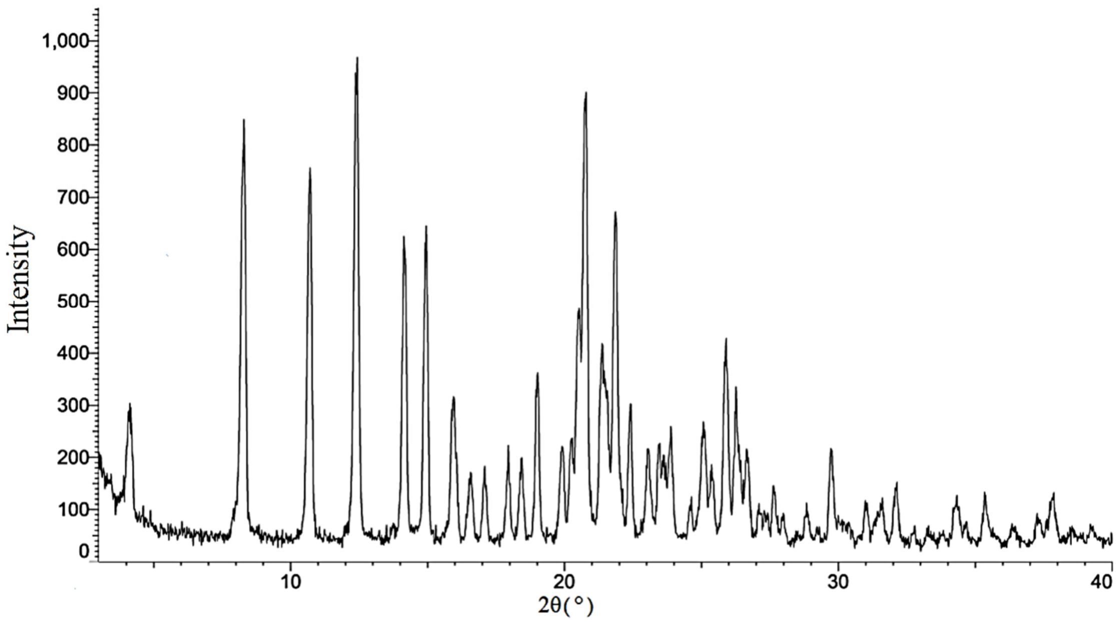
1,000
900
800
700
600
500
400
300
200
100
0
Intensity
10
20
30
40
2θ(°)

FIG. 1

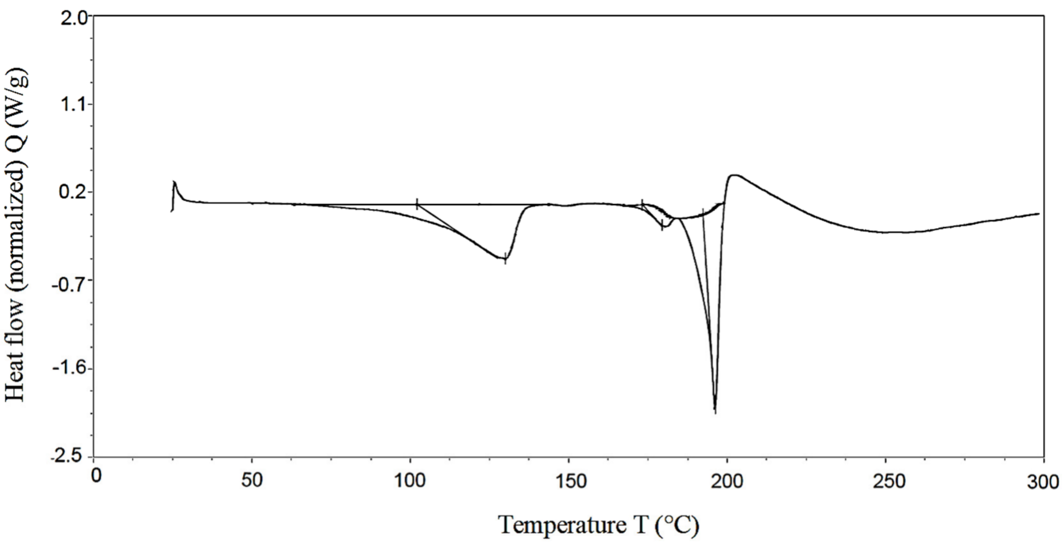
2.0
1.1
0.2
-0.7
-1.6
-2.5
Heat flow (normalized) Q (W/g)
0
50
100
150
200
250
300
Temperature T (°C)

FIG. 2

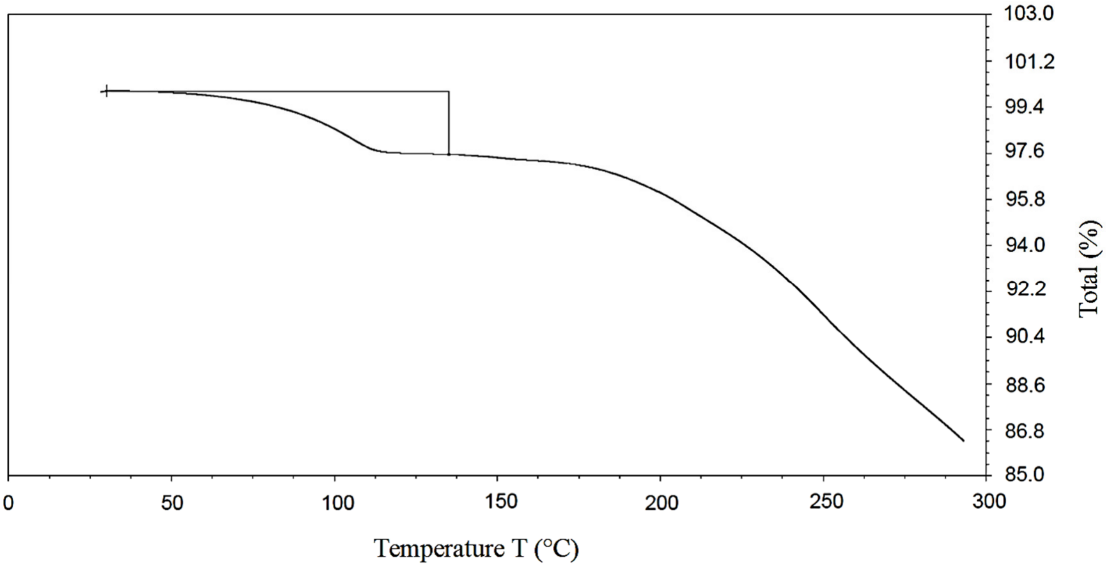
103.0
101.2
99.4
97.6
95.8
94.0
92.2
90.4
88.6
86.8
85.0
Total (%)
0
50
100
150
200
250
300
Temperature T (°C)

FIG. 3

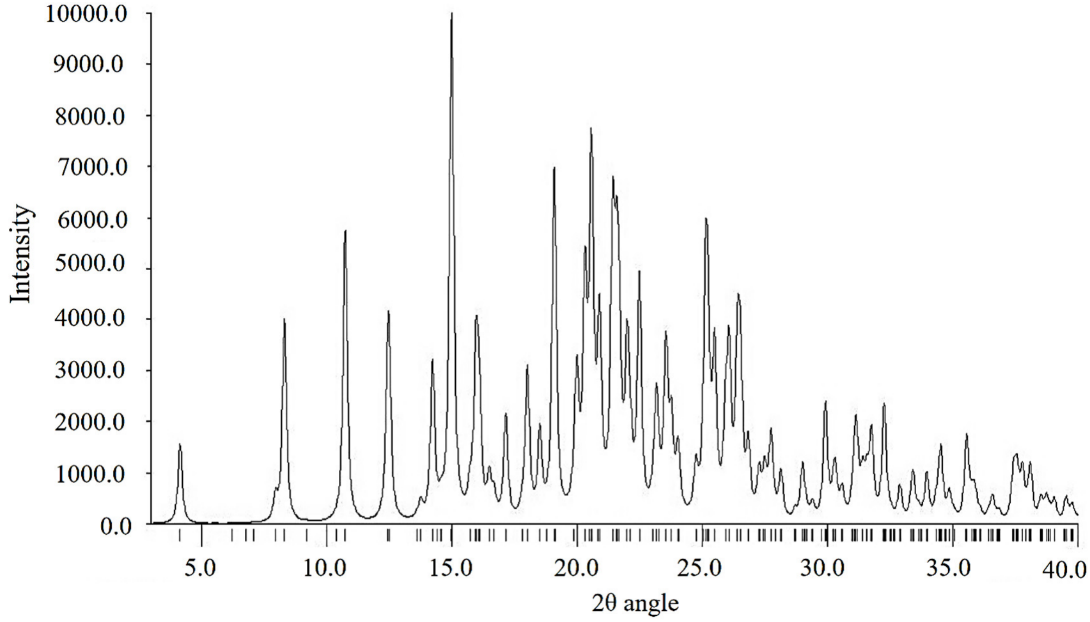
10000.0
9000.0
8000.0
7000.0
6000.0
5000.0
4000.0
3000.0
2000.0
1000.0
0.0
Intensity
5.0
10.0
15.0
20.0
25.0
30.0
35.0
40.0
2θ angle

FIG. 4

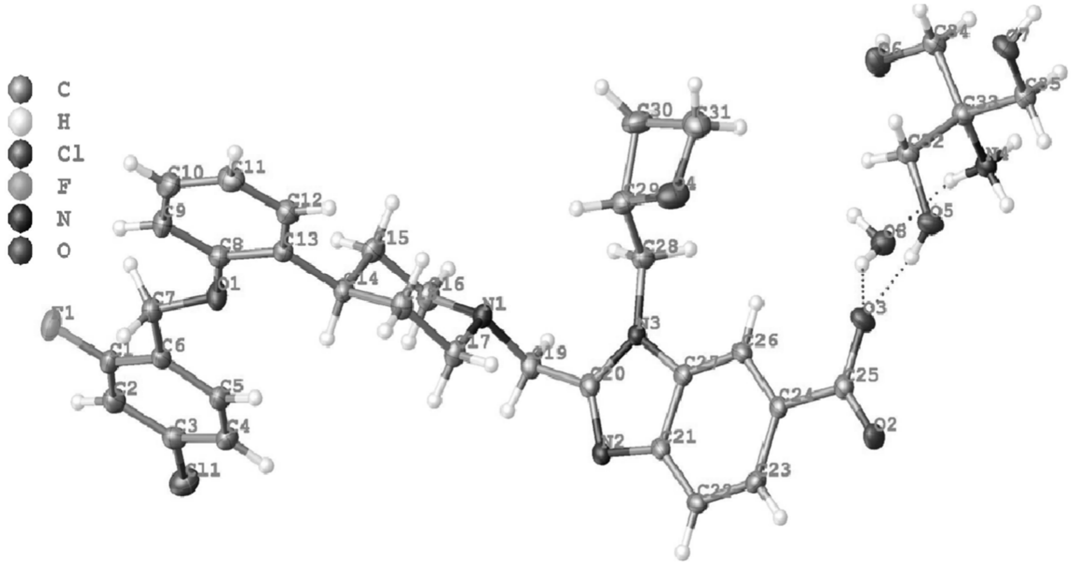

FIG. 5

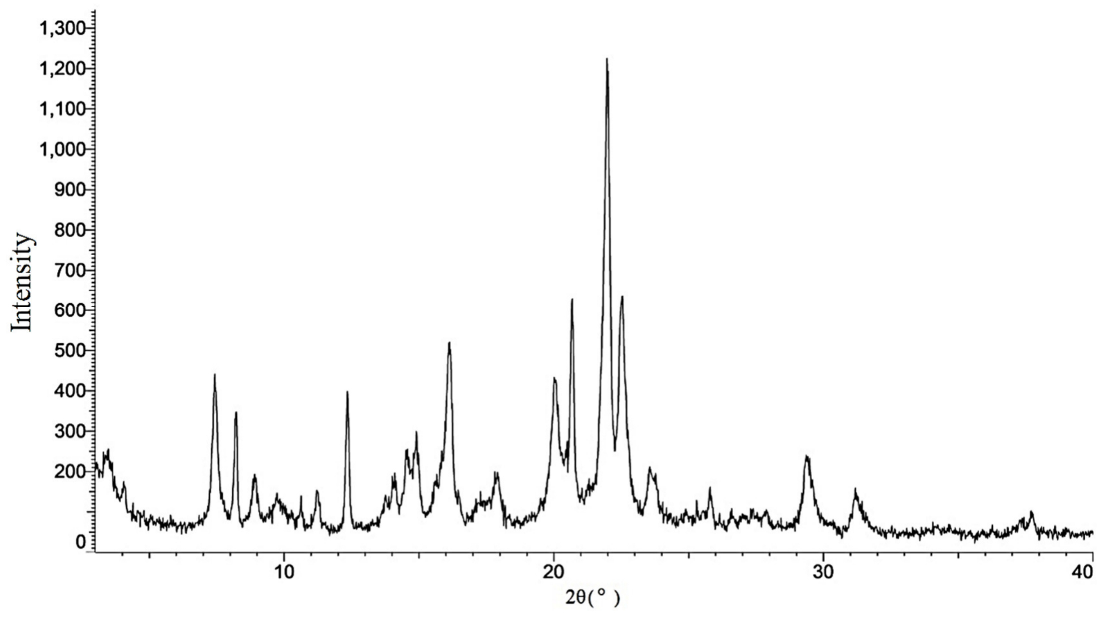
Intensity
1,300
1,200
1,100
1,000
900
800
700
600
500
400
300
200
0
10
20
30
40
2θ(° )

FIG. 6

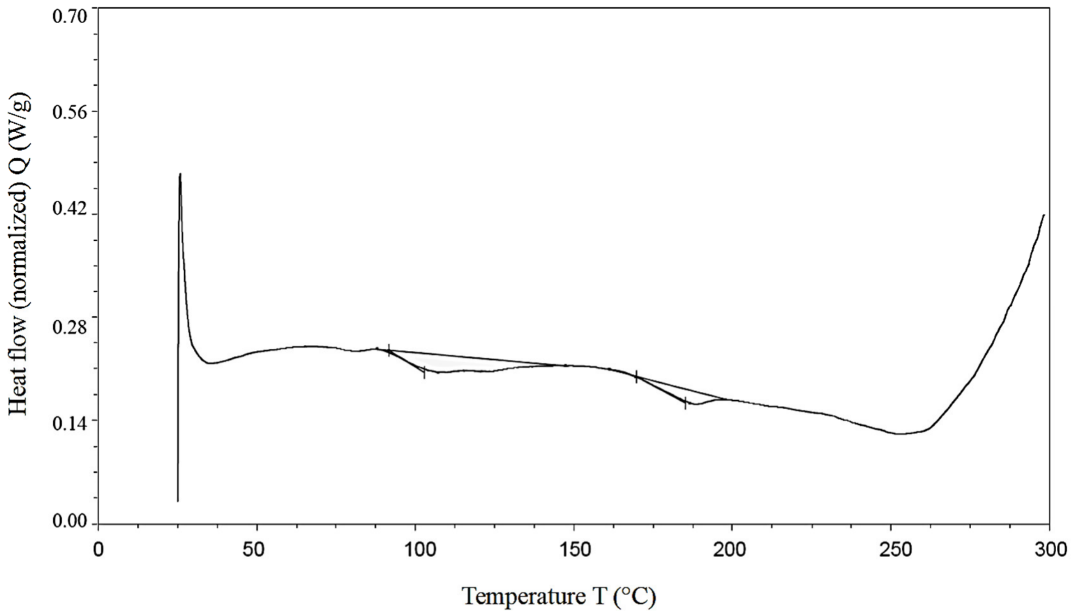
0.70
0.56
0.42
0.28
0.14
0.00
Heat flow (normalized) Q (W/g)
0
50
100
150
200
250
300
Temperature T (°C)

FIG. 7

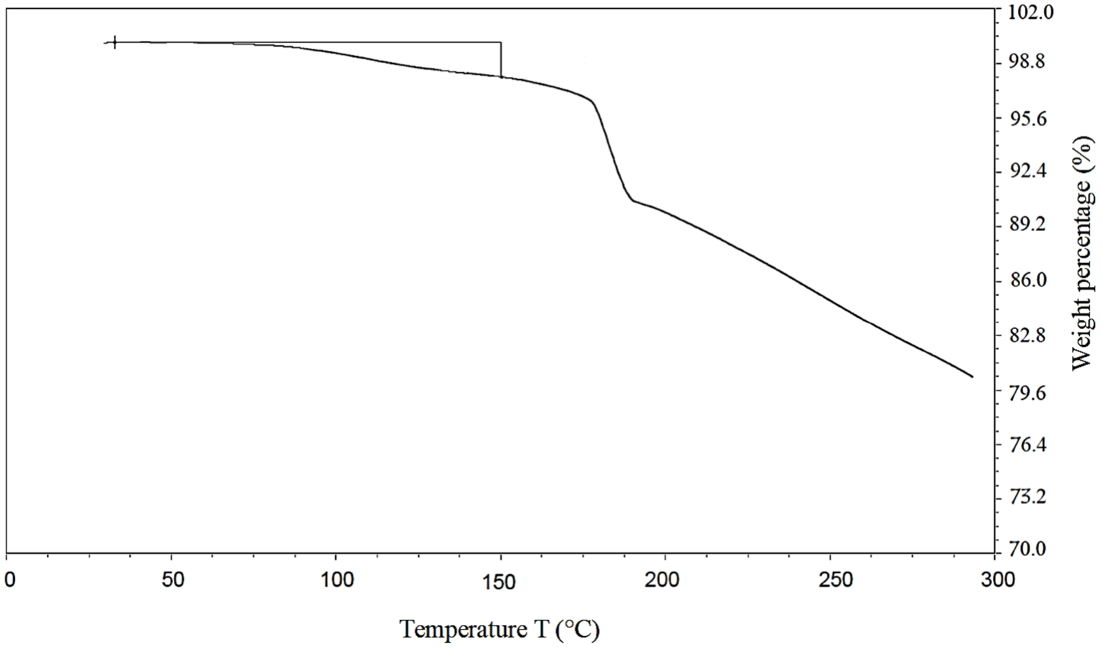
102.0
98.8
95.6
92.4
89.2
86.0
82.8
79.6
76.4
73.2
70.0
Weight percentage (%)
0
50
100
150
200
250
300
Temperature T (°C)

FIG. 8

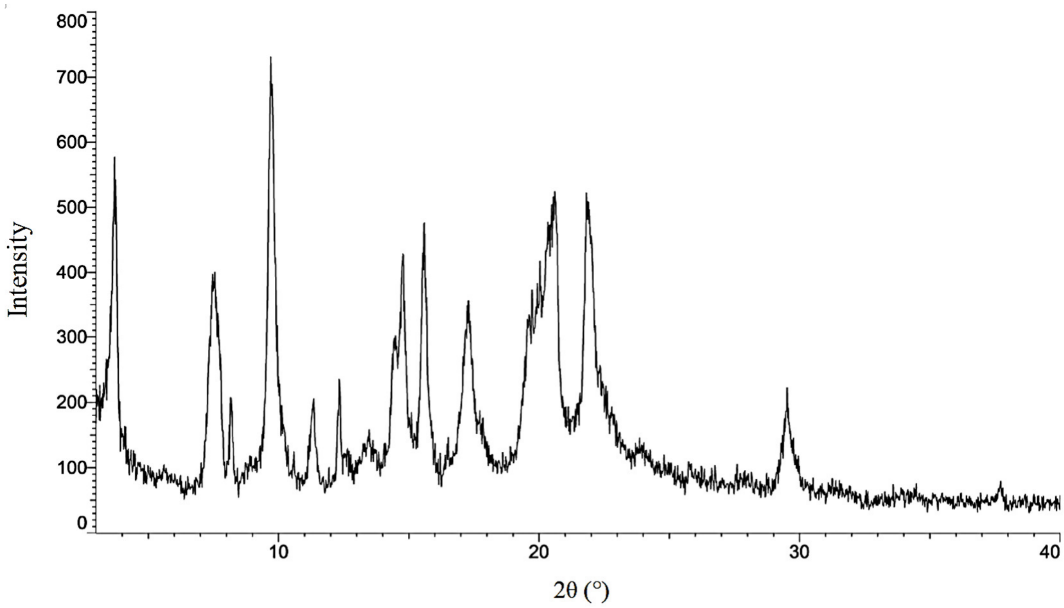
Intensity
800
700
600
500
400
300
200
100
0
10
20
30
40
2θ (°)

FIG. 9

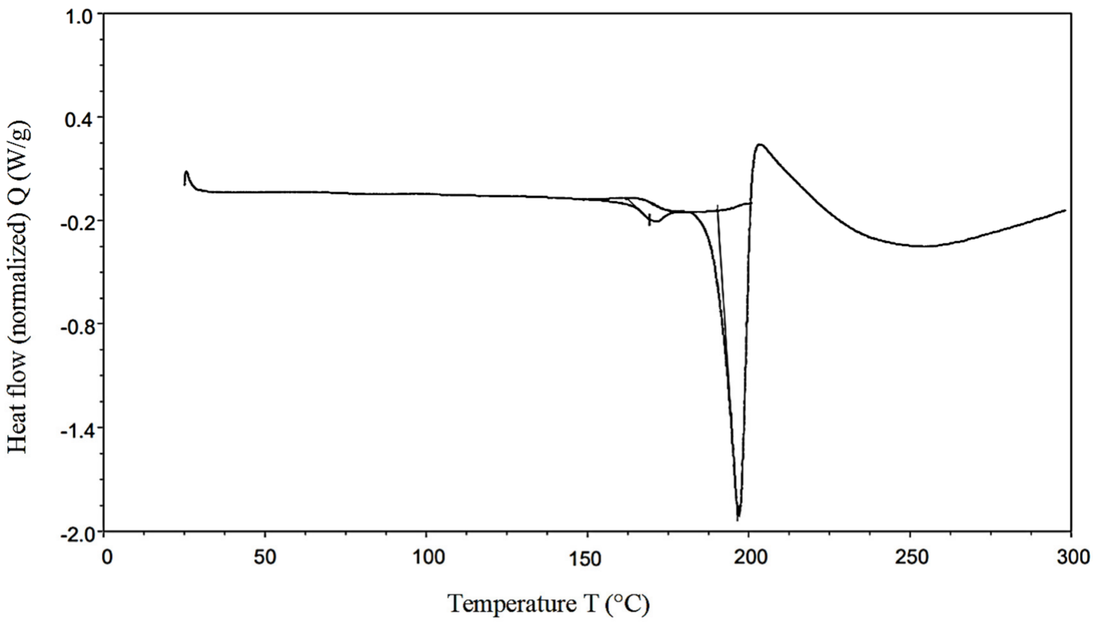
Heat flow (normalized) Q (W/g)
1.0
0.4
-0.2
-0.8
-1.4
-2.0
0
50
100
150
200
250
300
Temperature T (°C)

FIG. 10

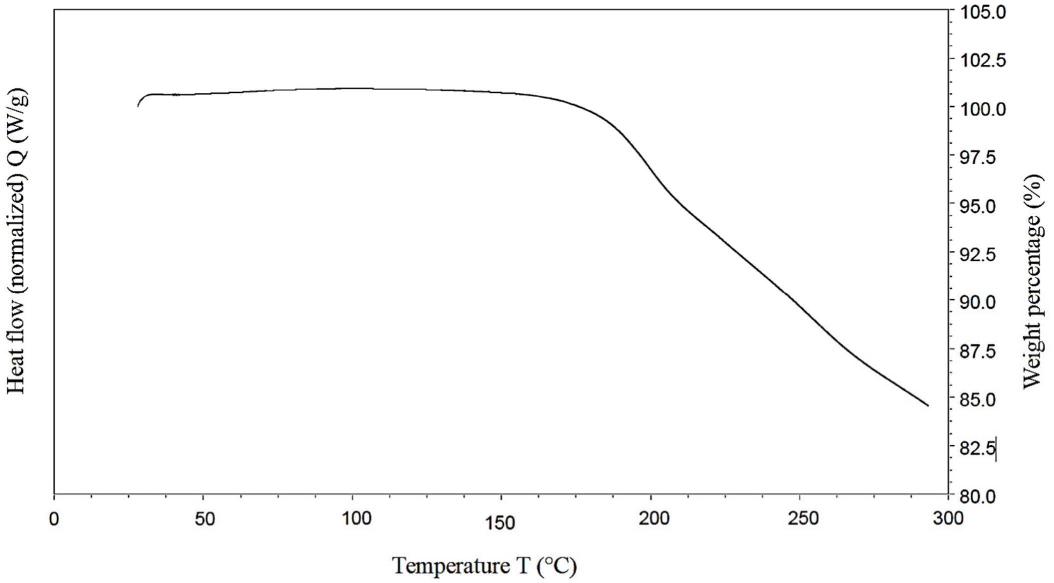
Heat flow (normalized) Q (W/g)
Weight percentage (%)
Temperature T (°C)
105.0
102.5
100.0
97.5
95.0
92.5
90.0
87.5
85.0
82.5
80.0
0
50
100
150
200
250
300

FIG. 11

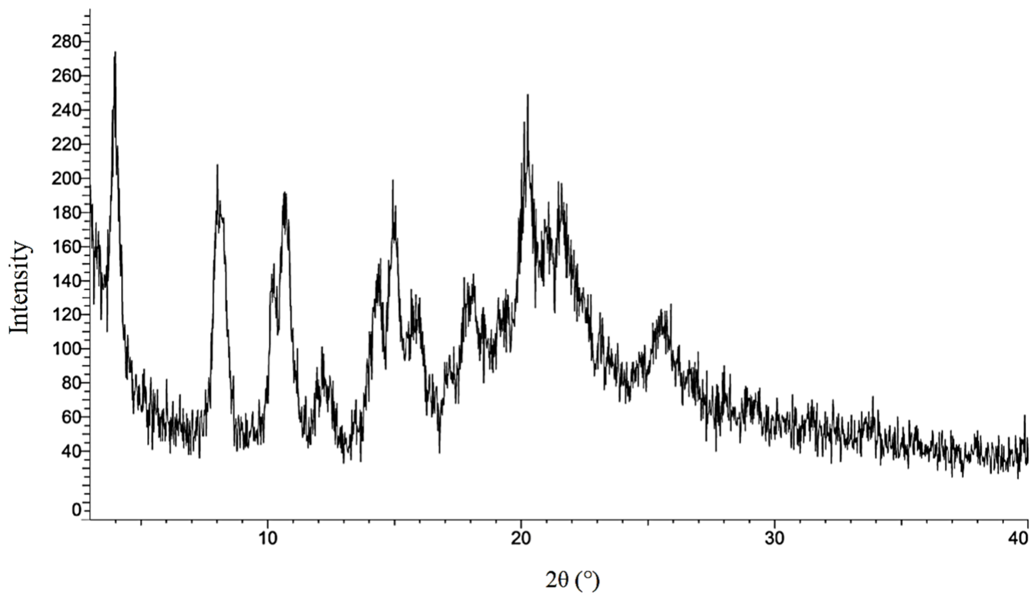
Intensity
2θ (°)
280
260
240
220
200
180
160
140
120
100
80
60
40
0
10
20
30
40

FIG. 12

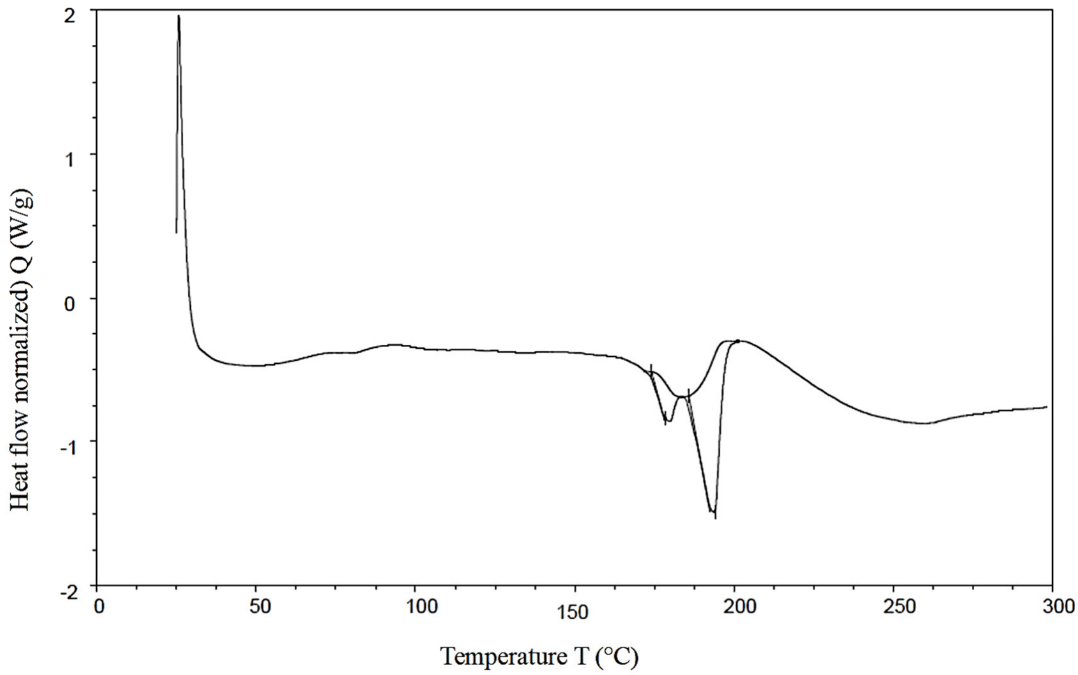
2
1
0
-1
-2
Heat flow normalized) Q (W/g)
0
50
100
150
200
250
300
Temperature T (°C)

FIG. 13

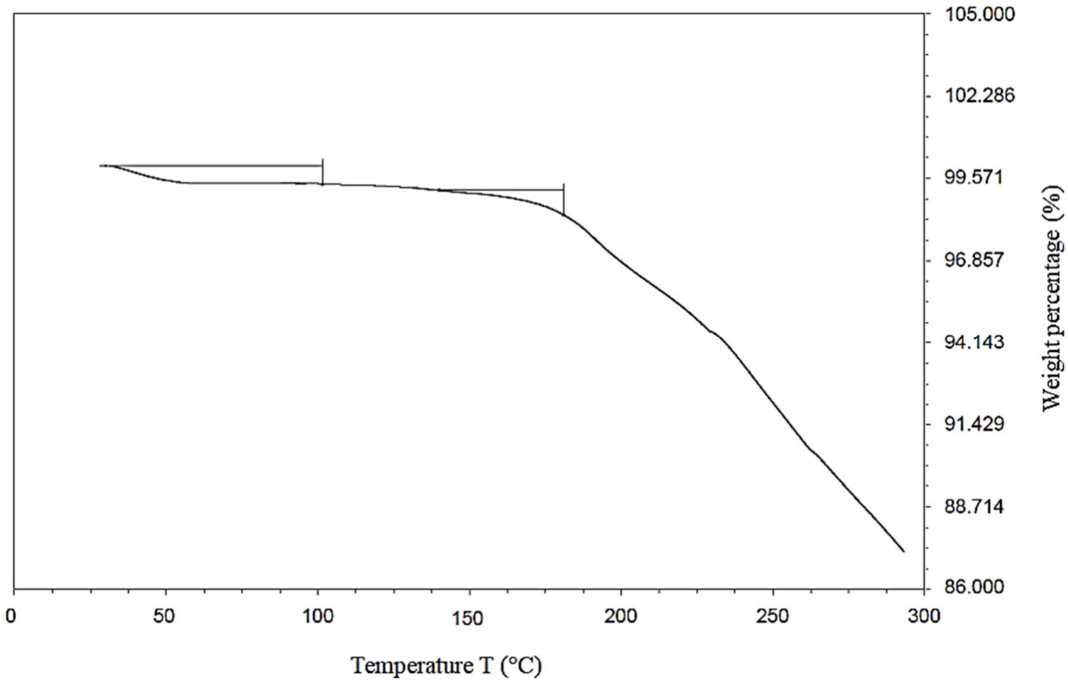
105.000
102.286
99.571
96.857
94.143
91.429
88.714
86.000
Weight percentage (%)
0
50
100
150
200
250
300
Temperature T (°C)

FIG. 14

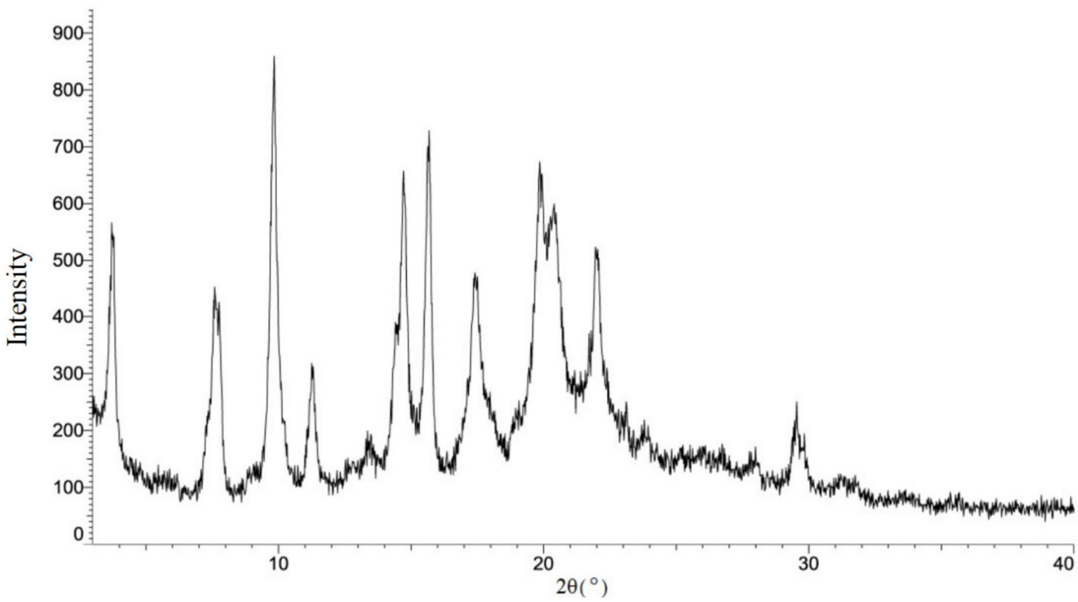
Intensity
900
800
700
600
500
400
300
200
100
0
10
20
30
40
2θ(°)

FIG. 15

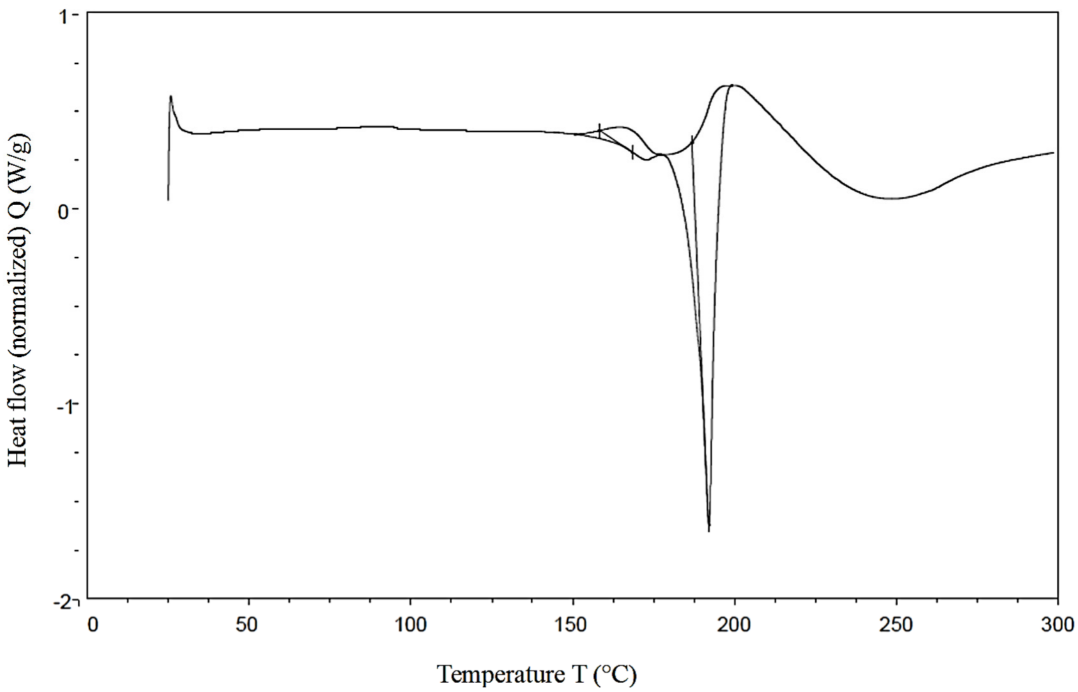
Heat flow (normalized) Q (W/g)
1
0
-1
-2
0
50
100
150
200
250
300
Temperature T (°C)

FIG. 16

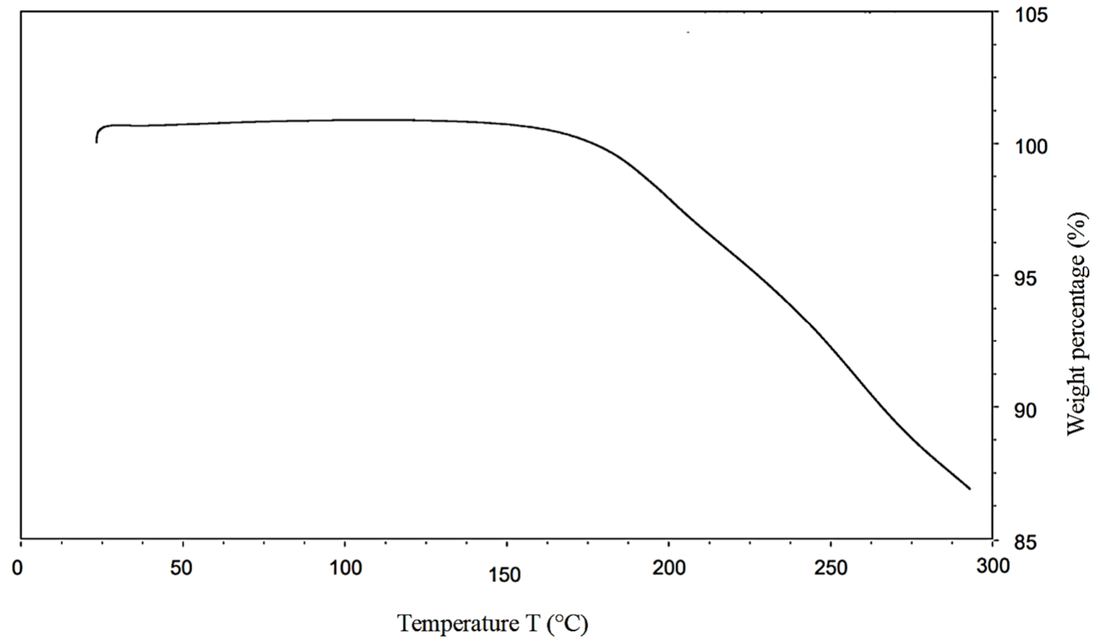
105
100
95
90
85
Weight percentage (%)
0
50
100
150
200
250
300
Temperature T (°C)

FIG. 17

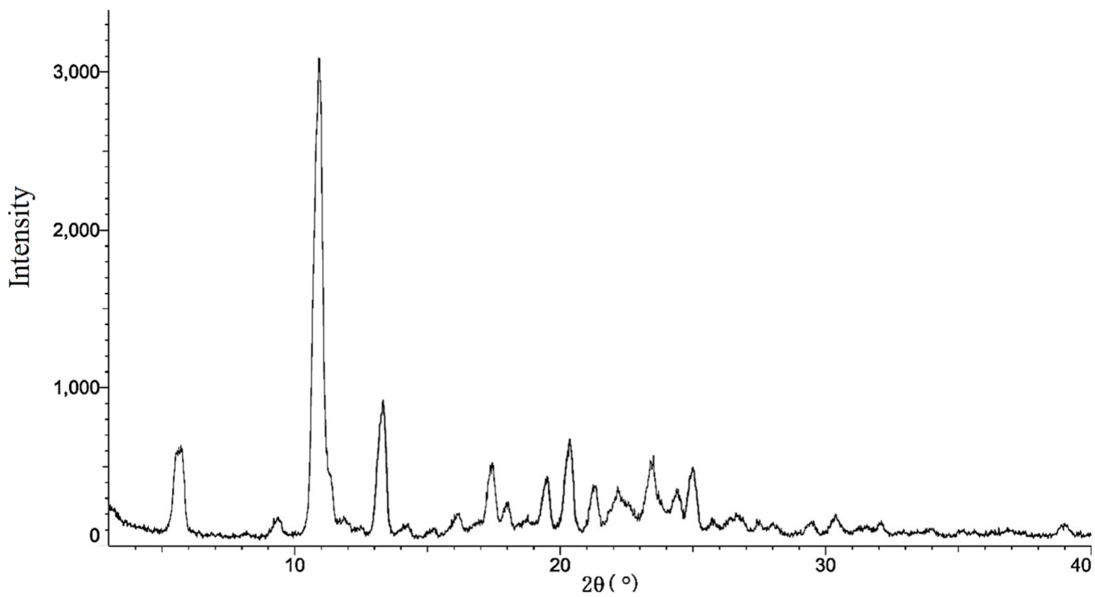
3,000
2,000
1,000
0
Intensity
10
20
30
40
2θ(°)

FIG. 18

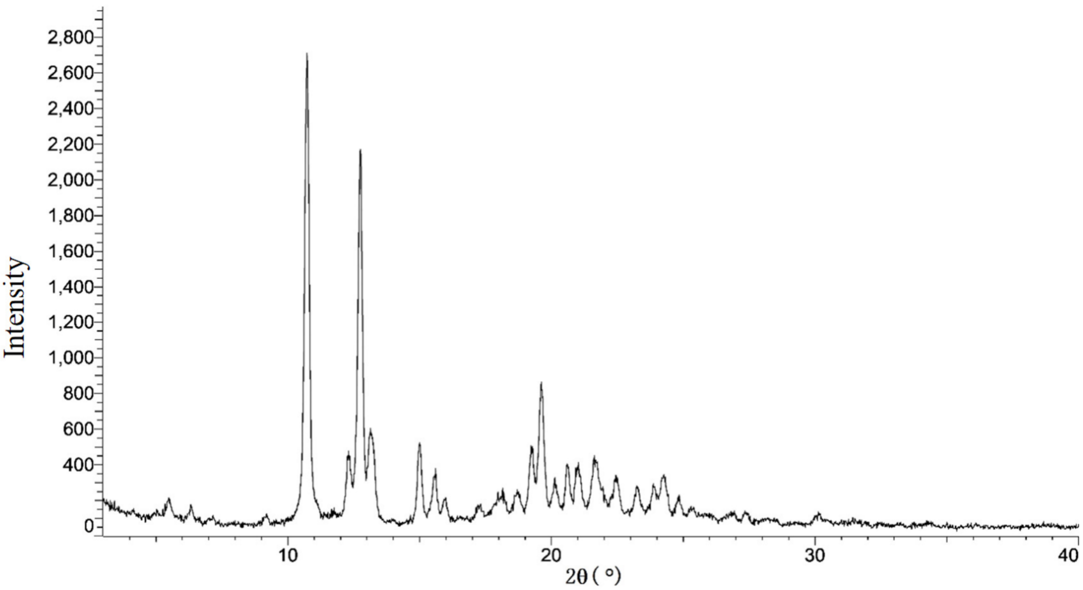
Intensity
2,800
2,600
2,400
2,200
2,000
1,800
1,600
1,400
1,200
1,000
800
600
400
0
10
20
30
40
2θ(°)

FIG. 19

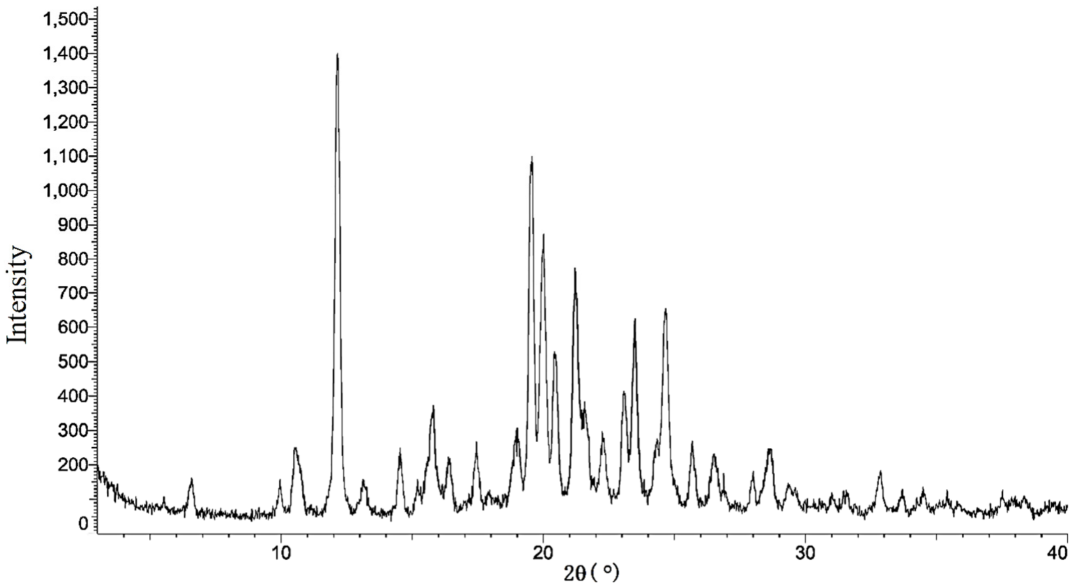
Intensity
1,500
1,400
1,300
1,200
1,100
1,000
900
800
700
600
500
400
300
200
0
10
20
30
40
2θ(°)

FIG. 20

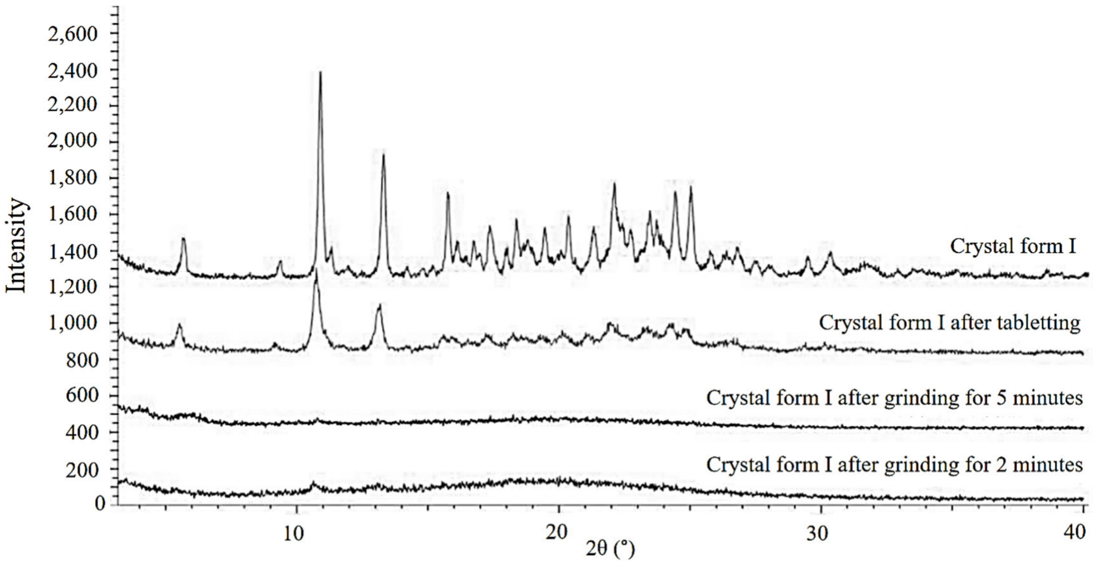


FIG. 21

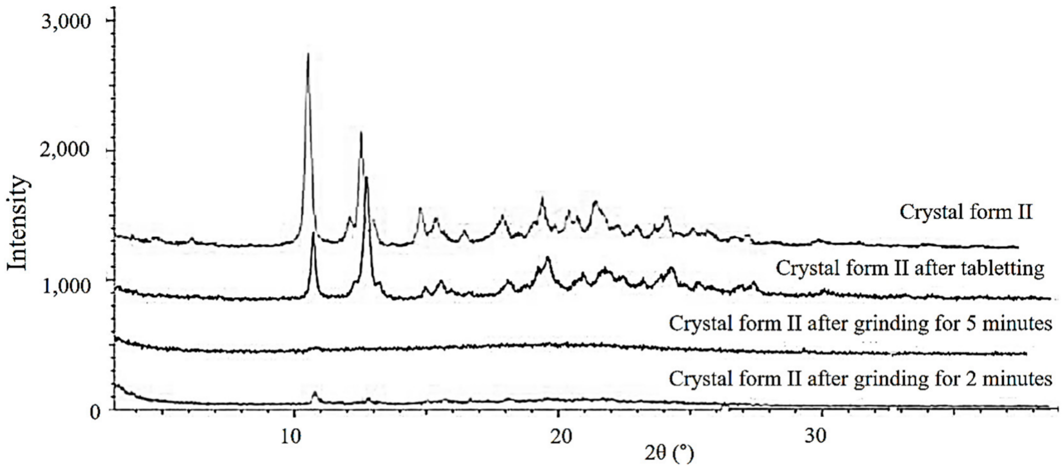


FIG. 22

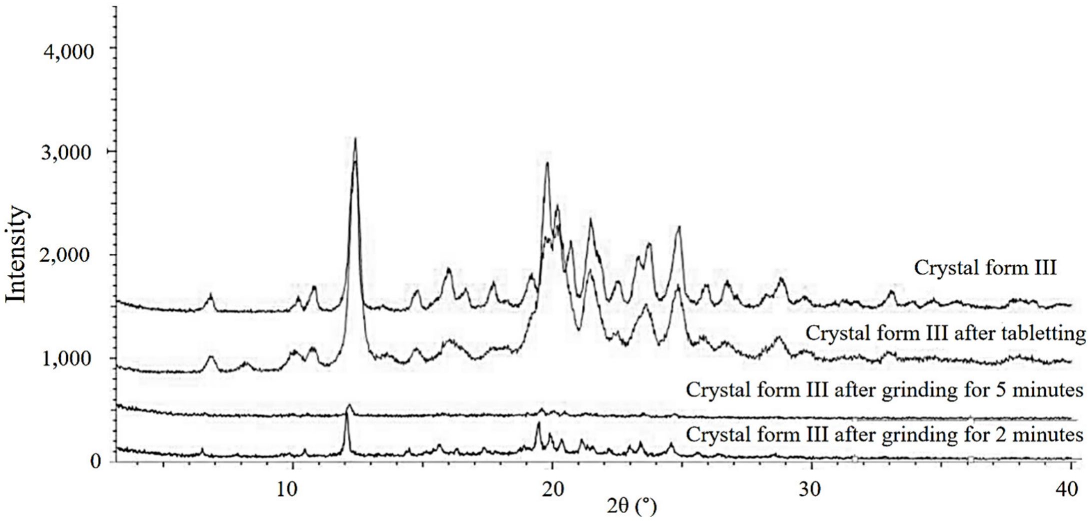


FIG. 23

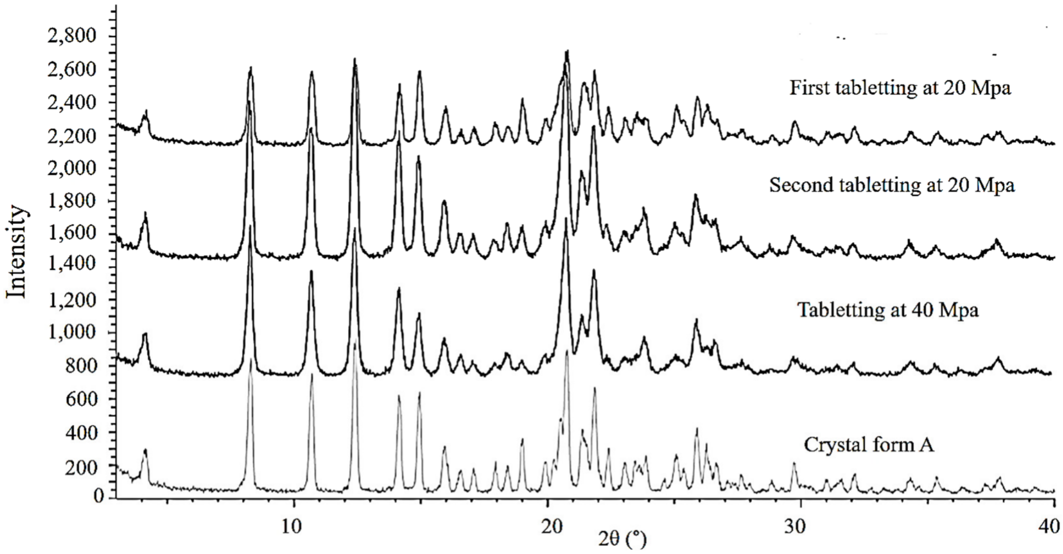
2,800
2,600
2,400
2,200
2,000
1,800
1,600
1,400
1,200
1,000
800
600
400
200
0
Intensity
First tabletting at 20 Mpa
Second tabletting at 20 Mpa
Tabletting at 40 Mpa
Crystal form A
10
20
30
40
2θ (°)

FIG. 24

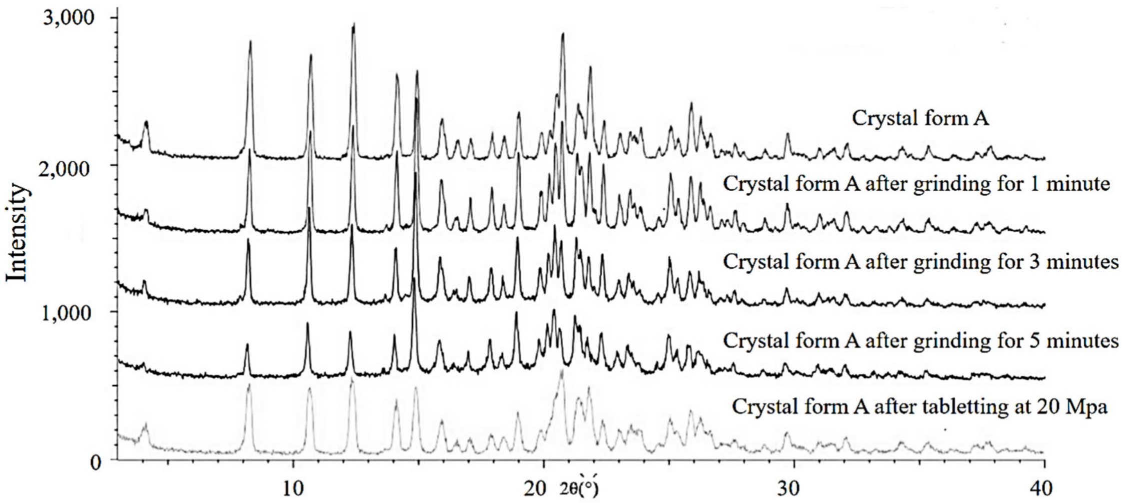
3,000
2,000
1,000
0
Intensity
Crystal form A
Crystal form A after grinding for 1 minute
Crystal form A after grinding for 3 minutes
Crystal form A after grinding for 5 minutes
Crystal form A after tabletting at 20 Mpa
10
20
30
40
2θ(°)

FIG. 25

**INTERNATIONAL SEARCH REPORT**

International application No.
**PCT/CN2024/088887**

**A. CLASSIFICATION OF SUBJECT MATTER**

C07D 405/14(2006.01)i； A61K 31/4439(2006.01)i； A61P 3/00(2006.01)i； A61P 1/16(2006.01)i； A61P 3/10(2006.01)i； A61P 3/06(2006.01)i； A61P 3/04(2006.01)i； A61P 9/10(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, ENTXTC, DWPI, WPABS, CNKI, ISI_web of Scicence, STN: 苏州闻泰医药科技有限公司, 李本, 苯并咪唑, 哌啶, 2-氨基-2-(羟甲基)-1,3-丙二醇, 晶体, 溶剂合物, 糖尿病, 高血糖, benzoimidazol, piperidin, 2-amino-2-(hydroxymethyl)-1,3-propanediol, crystal, solvate, diabetes, hyperglycemia, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| Y | CN 114591308 A (SUZHOU WENTAI MEDICINE TECHNOLOGY CO., LTD.) 07 June 2022 (2022-06-07)<br>description, paragraphs 91, 94, 95 and 101 | 1-12 |
| Y | CN 115175893 A (PFIZER INC.) 11 October 2022 (2022-10-11)<br>claim 26, and description, paragraph 16 | 1-12 |
| Y | CN 110325530 A (PFIZER INC.) 11 October 2019 (2019-10-11)<br>description, paragraphs 721 and 722 | 1-12 |
| A | CN 112533674 A (PFIZER INC.) 19 March 2021 (2021-03-19)<br>description, paragraph 342 | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 July 2024** | **04 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/088887**

| Patent document cited in search report | | | Publication date (day/month/year) | Patent family member(s) | | | Publication date (day/month/year) |
|---|---|---|---|---|---|---|---|
| CN | 114591308 | A | 07 June 2022 | WO | 2022116693 | A1 | 09 June 2022 |
| | | | | EP | 4212527 | A1 | 19 July 2023 |
| | | | | US | 11773087 | B2 | 03 October 2023 |
| | | | | JP | 7456699 | B2 | 27 March 2024 |
| | | | | EP | 4212527 | A4 | 27 December 2023 |
| | | | | CN | 114591308 | B | 08 March 2024 |
| CN | 115175893 | A | 11 October 2022 | BR | 112022010599 | A2 | 16 August 2022 |
| | | | | AU | 2020402177 | A1 | 16 June 2022 |
| | | | | TW | 202136247 | A | 01 October 2021 |
| | | | | TWI | 809334 | B | 21 July 2023 |
| | | | | WO | 2021116874 | A1 | 17 June 2021 |
| | | | | EP | 4073028 | A1 | 19 October 2022 |
| | | | | KR | 20220112811 | A | 11 August 2022 |
| | | | | MX | 2022007105 | A | 11 July 2022 |
| | | | | US | 2023045419 | A1 | 09 February 2023 |
| | | | | ZA | 202207589 | B | 26 April 2023 |
| | | | | JP | 2021091683 | A | 17 June 2021 |
| | | | | CA | 3163979 | A1 | 17 June 2021 |
| CN | 110325530 | A | 11 October 2019 | US | 2019119255 | A1 | 25 April 2019 |
| | | | | US | 10669259 | B2 | 02 June 2020 |
| | | | | US | 2024034725 | A1 | 01 February 2024 |
| | | | | MX | 2019007077 | A | 01 August 2019 |
| | | | | IL | 267288 | A | 29 August 2019 |
| | | | | IL | 267288 | B | 30 June 2021 |
| | | | | PH | 12019501360 | A1 | 10 February 2020 |
| | | | | US | 2020255406 | A1 | 13 August 2020 |
| | | | | US | 10851081 | B2 | 01 December 2020 |
| | | | | TW | 201835066 | A | 01 October 2018 |
| | | | | TWI | 713809 | B | 21 December 2020 |
| | | | | US | 2018170908 | A1 | 21 June 2018 |
| | | | | US | 10208019 | B2 | 19 February 2019 |
| | | | | MY | 195385 | A | 18 January 2023 |
| | | | | AU | 2017374860 | A1 | 20 June 2019 |
| | | | | AU | 2017374860 | B2 | 02 September 2021 |
| | | | | EP | 3555064 | A1 | 23 October 2019 |
| | | | | EP | 3555064 | B1 | 23 November 2022 |
| | | | | EP | 3555064 | B9 | 01 March 2023 |
| | | | | KR | 20190094433 | A | 13 August 2019 |
| | | | | KR | 102314286 | B1 | 21 October 2021 |
| | | | | JP | 6637641 | B1 | 29 January 2020 |
| | | | | JP | 2020511411 | A | 16 April 2020 |
| | | | | FI | 3555064 | T3 | 31 January 2023 |
| | | | | JP | 2020063287 | A | 23 April 2020 |
| | | | | JP | 6982054 | B2 | 17 December 2021 |
| | | | | US | 2021047298 | A1 | 18 February 2021 |
| | | | | US | 11512070 | B2 | 29 November 2022 |
| | | | | BR | 112019012211 | A2 | 12 November 2019 |
| | | | | KR | 20210127782 | A | 22 October 2021 |
| | | | | KR | 102466418 | B1 | 14 November 2022 |
| | | | | US | 2023124938 | A1 | 20 April 2023 |

Form PCT/ISA/210 (patent family annex) (July 2022)

**INTERNATIONAL SEARCH REPORT**
**Information on patent family members**

International application No.
**PCT/CN2024/088887**

| Patent document cited in search report | Publication date (day/month/year) | Patent family member(s) | Publication date (day/month/year) |
|---|---|---|---|
| | | US 11802121 B2 | 31 October 2023 |
| | | ES 2934789 T3 | 27 February 2023 |
| | | ES 2934789 T9 | 26 April 2023 |
| | | WO 2018109607 A1 | 21 June 2018 |
| | | CA 2988721 C | 05 September 2023 |
| | | EA 037318 B1 | 11 March 2021 |
| CN 112533674 A | 19 March 2021 | US 2021163455 A1 | 03 June 2021 |
| | | ES 2949954 T3 | 04 October 2023 |
| | | ES 2949954 T9 | 23 November 2023 |
| | | EP 3806955 A1 | 21 April 2021 |
| | | EP 3806955 B1 | 31 May 2023 |
| | | EP 3806955 B9 | 23 August 2023 |
| | | MX 2020013625 A | 04 January 2022 |
| | | IL 279300 A | 31 January 2021 |
| | | IL 279300 B2 | 01 November 2023 |
| | | PH 12020552069 A1 | 31 May 2021 |
| | | KR 20210019529 A | 22 February 2021 |
| | | KR 102542199 B1 | 13 June 2023 |
| | | US 2019382387 A1 | 19 December 2019 |
| | | US 10683281 B2 | 16 June 2020 |
| | | US 2019382388 A1 | 19 December 2019 |
| | | US 10676465 B2 | 09 June 2020 |
| | | US 2024059679 A1 | 22 February 2024 |
| | | JP 6916968 B1 | 11 August 2021 |
| | | JP 2021521265 A | 26 August 2021 |
| | | RU 2769715 C1 | 05 April 2022 |
| | | US 2019382384 A1 | 19 December 2019 |
| | | US 10934279 B2 | 02 March 2021 |
| | | AU 2019285491 A1 | 17 December 2020 |
| | | AU 2019285491 B2 | 11 November 2021 |
| | | EP 4219487 A1 | 02 August 2023 |
| | | WO 2019239319 A1 | 19 December 2019 |
| | | TW 202015679 A | 01 May 2020 |
| | | TWI 707683 B | 21 October 2020 |
| | | CA 3045644 A1 | 13 December 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023104355011
- CN 202110334388